# EUROPEAN PATENT APPLICATION

(11) **EP 4 286 520 A1**
(43) Date of publication of application: **06.12.2023**
(21) Application number: 22745326.3
(22) Date of filing: 28.01.2022
(51) Int. Cl.: C12N 15/13, C07K 16/18, A61K 39/395, A61P 31/00

(54) **ANTIGEN BINDING PROTEIN AND USE THEREOF**

(30) Priority: 29.01.2021 CN 202110124760
(71) Applicant: Minghui Pharmaceutical (Hangzhou) Limited, Hangzhou, Zhejiang 310018 (CN); Minghui Pharmaceutical (Shanghai) Limited, Pilot Free Trade Zone Pudong New Area Shanghai 201203 (CN)
(72) Inventor: CAO, Guoqing, Shanghai 201203 (CN); ZHU, Liyuan, Shanghai 201203 (CN); SHI, Junwei, Shanghai 201203 (CN)
(74) Representative: Weickmann & Weickmann PartmbB
(86) International application number: PCT/CN2022/074550
(87) International publication number: WO 2022/161454

(57) **Abstract**

An antigen binding protein and the use thereof. The antigen binding protein can compete with pembrolizumab for binding to the same or overlapping PD-1 epitope. The antigen binding protein can effectively inhibit the binding of human PD-L1 to human PD-1 with an EC₅₀ of approximately 2 µg/mL or less.

## Description

### Field of the Invention

The present application relates to the field of biomedicine, and specifically to an antigen binding protein and a use thereof.

### Background of the Invention

Human programmed cell death receptor-1 (PD-1) is one of the known major immune checkpoints which are expressed on the surface of activated T lymphocytes, and when binding to ligands PD-L1 (programmed cell death receptor-ligand 1) and PD-L2 (programmed cell death receptor-ligand 2), it can inhibit the activity of T lymphocytes as well as related cellular immune responses in vivo. PD-L1 is widely expressed in B, T lymphocytes and peripheral cells such as microvascular epithelial cells, as well as lung, liver, heart, and other tissue cells. Numerous studies have shown that, the interaction between PD-1 and PD-L1 is the main reason for the evasion of immune surveillance by PD-L1-expressing positive tumor cells. By blocking the negative regulation of PD-1/PD-L1 signaling pathway by tumor cells and activating the immune system, it can promote T cell-related tumor-specific cellular immune responses and treat tumors.

At present, anti-PD-1 antibodies also have the disadvantages of poor selectivity and low affinity. Therefore, it is necessary to develop a novel anti-PD-1 antibody with high affinity and great specificity for PD-1.

### Summary of the Invention

The present application provides an isolated antigen binding protein, which has one or more of the following properties: 1) binding to PD-1 with an EC₅₀ of about 11 µg/mL or less in ELISA assay; 2) inhibiting the binding of human PD-L1 to human PD-1 with an EC₅₀ of about 2 µg/mL or less in competitive ELISA assay; and 3) capable of binding to PD-1 of primates (e.g., human or monkeys).

In some embodiments, the isolated antigen binding protein binds to the same or overlapping PD-1 epitope with a reference antibody.

In some embodiments, the isolated antigen binding protein competes with the reference antibody for binding to PD-1.

In some embodiments, the binding affinity of the isolated antigen binding protein for PD-1 is substantially the same as the binding affinity of the reference antibody for PD-1.

In some embodiments, the reference antibody includes a HCDR3, and the HCDR3 includes an amino acid sequence as shown in SEQ ID NO: 128.

In some embodiments, the reference antibody includes a HCDR2, and the HCDR2 includes an amino acid sequence as shown in SEQ ID NO: 127.

In some embodiments, the reference antibody includes a HCDR1, and the HCDR1 includes an amino acid sequence as shown in SEQ ID NO: 126.

In some embodiments, the reference antibody includes a heavy chain variable region VH, and the VH includes the HCDR1, HCDR2, and HCDR3, the HCDR3 includes an amino acid sequence as shown in SEQ ID NO: 128; the HCDR2 includes an amino acid sequence as shown in SEQ ID NO: 127; and the HCDR1 includes an amino acid sequence as shown in SEQ ID NO: 126.

In some embodiments, the VH of the reference antibody includes an amino acid sequence as shown in SEQ ID NO: 132.

In some embodiments, the reference antibody includes a LCDR3, and the LCDR3 includes an amino acid sequence as shown in SEQ ID NO: 131.

In some embodiments, the reference antibody includes a LCDR2, and the LCDR2 includes an amino acid sequence as shown in SEQ ID NO: 130.

In some embodiments, the reference antibody includes a LCDR1, and the LCDR1 includes an amino acid sequence as shown in SEQ ID NO: 129.

In some embodiments, the reference antibody includes a light chain variable region VL, and the VL includes the LCDR1, LCDR2, and LCDR3, the LCDR3 includes an amino acid sequence as shown in SEQ ID NO: 131; the LCDR2 includes an amino acid sequence as shown in SEQ ID NO: 130; and the LCDR1 includes an amino acid sequence as shown in SEQ ID NO: 129.

In some embodiments, the VL of the reference antibody includes an amino acid sequence as shown in SEQ ID NO: 133.

In some embodiments, the reference antibody includes pembrolizumab or an antigen binding fragment thereof.

In some embodiments, the isolated antigen binding protein includes a HCDR3, and the HCDR3 includes an amino acid sequence as shown in SEQ ID NO: 138.

In some embodiments, the isolated antigen binding protein includes a HCDR3, and the HCDR3 includes an amino acid sequence as shown in SEQ ID NO: 142.

In some embodiments, the isolated antigen binding protein includes a HCDR3, and the HCDR3 includes an amino acid sequence as shown in any one of SEQ ID NOs: 3, 55, 56, 57, 58, 59, 60, 61, 62, 63, 64, 65, 66, and 67.

In some embodiments, the isolated antigen binding protein includes a HCDR2, and the HCDR2 includes an amino acid sequence as shown in SEQ ID NO: 137.

In some embodiments, the isolated antigen binding protein includes a HCDR2, and the HCDR2 includes an amino acid sequence as shown in SEQ ID NO: 141.

In some embodiments, the isolated antigen binding protein includes a HCDR2, and the HCDR2 includes an amino acid sequence as shown in any one of SEQ ID NOs: 2, 42, 43, 44, 45, 46, 47, 48, 49, 50, 51, 52, 53, and 54.

In some embodiments, the isolated antigen binding protein includes a HCDR1, and the HCDR1 includes an amino acid sequence as shown in SEQ ID NO: 136.

In some embodiments, the isolated antigen binding protein includes a HCDR1, and the HCDR1 includes an amino acid sequence as shown in SEQ ID NO: 140.

In some embodiments, the isolated antigen binding protein includes a HCDR1, and the HCDR1 includes an amino acid sequence as shown in any one of SEQ ID NOs: 1, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 41.

In some embodiments, the isolated antigen binding protein includes a heavy chain variable region VH, and the VH includes the HCDR1, HCDR2, and HCDR3, the HCDR3 includes an amino acid sequence as shown in SEQ ID NO: 138; the HCDR2 includes an amino acid sequence as shown in SEQ ID NO: 137; and the HCDR1 includes an amino acid sequence as shown in SEQ ID NO: 136.

In some embodiments, the isolated antigen binding protein includes a heavy chain variable region VH, and the VH includes the HCDR1, HCDR2, and HCDR3, the HCDR3 includes an amino acid sequence as shown in SEQ ID NO: 142; the HCDR2 includes an amino acid sequence as shown in SEQ ID NO: 141; and the HCDR1 includes an amino acid sequence as shown in SEQ ID NO: 140.

In some embodiments, the VH of the isolated antigen binding protein includes the HCDR1, HCDR2, and HCDR3, and the HCDR3 includes an amino acid sequence as shown in any one of SEQ ID NOs: 3, 55, 56, 57, 58, 59, 60, 61, 62, 63, 64, 65, 66, and 67; the HCDR2 includes an amino acid sequence as shown in any one of SEQ ID NOs: 2, 42, 43, 44, 45, 46, 47, 48, 49, 50, 51, 52, 53, and 54; and the HCDR1 includes an amino acid sequence as shown in any one of SEQ ID NOs: 1, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 41.

In some embodiments, the HCDR1, HCDR2, and HCDR3 of the isolated antigen binding protein include any one group of amino acid sequences selected from:
1) HCDR1: SEQ ID NO: 1, HCDR2: SEQ ID NO: 2, and HCDR3: SEQ ID NO: 3;
2) HCDR1: SEQ ID NO: 1, HCDR2: SEQ ID NO: 42, and HCDR3: SEQ ID NO: 55;
3) HCDR1: SEQ ID NO: 4, HCDR2: SEQ ID NO: 43, and HCDR3: SEQ ID NO: 3;
4) HCDR1: SEQ ID NO: 5, HCDR2: SEQ ID NO: 44, and HCDR3: SEQ ID NO: 3;
5) HCDR1: SEQ ID NO: 6, HCDR2: SEQ ID NO: 45, and HCDR3: SEQ ID NO: 3;
6) HCDR1: SEQ ID NO: 7, HCDR2: SEQ ID NO: 46, and HCDR3: SEQ ID NO: 3;
7) HCDR1: SEQ ID NO: 8, HCDR2: SEQ ID NO: 47, and HCDR3: SEQ ID NO: 3;
8) HCDR1: SEQ ID NO: 9, HCDR2: SEQ ID NO: 46, and HCDR3: SEQ ID NO: 3;
9) HCDR1: SEQ ID NO: 10, HCDR2: SEQ ID NO: 45, and HCDR3: SEQ ID NO: 3;
10) HCDR1: SEQ ID NO: 11, HCDR2: SEQ ID NO: 44, and HCDR3: SEQ ID NO: 3;
11) HCDR1: SEQ ID NO: 12, HCDR2: SEQ ID NO: 43, and HCDR3: SEQ ID NO: 3;
12) HCDR1: SEQ ID NO: 13, HCDR2: SEQ ID NO: 48, and HCDR3: SEQ ID NO: 3;
13) HCDR1: SEQ ID NO: 14, HCDR2: SEQ ID NO: 45, and HCDR3: SEQ ID NO: 3;
14) HCDR1: SEQ ID NO: 15, HCDR2: SEQ ID NO: 47, and HCDR3: SEQ ID NO: 3;
15) HCDR1: SEQ ID NO: 16, HCDR2: SEQ ID NO: 43, and HCDR3: SEQ ID NO: 3;
16) HCDR1: SEQ ID NO: 17, HCDR2: SEQ ID NO: 49, and HCDR3: SEQ ID NO: 3;
17) HCDR1: SEQ ID NO: 18, HCDR2: SEQ ID NO: 46, and HCDR3: SEQ ID NO: 3;
18) HCDR1: SEQ ID NO: 19, HCDR2: SEQ ID NO: 46, and HCDR3: SEQ ID NO: 3;
19) HCDR1: SEQ ID NO: 20, HCDR2: SEQ ID NO: 50, and HCDR3: SEQ ID NO: 56;
20) HCDR1: SEQ ID NO: 21, HCDR2: SEQ ID NO: 46, and HCDR3: SEQ ID NO: 57;
21) HCDR1: SEQ ID NO: 22, HCDR2: SEQ ID NO: 46, and HCDR3: SEQ ID NO: 3;
22) HCDR1: SEQ ID NO: 9, HCDR2: SEQ ID NO: 51, and HCDR3: SEQ ID NO: 3;
23) HCDR1: SEQ ID NO: 9, HCDR2: SEQ ID NO: 46, and HCDR3: SEQ ID NO: 58;
24) HCDR1: SEQ ID NO: 23, HCDR2: SEQ ID NO: 46, and HCDR3: SEQ ID NO: 3;
25) HCDR1: SEQ ID NO: 24, HCDR2: SEQ ID NO: 46, and HCDR3: SEQ ID NO: 59;
26) HCDR1: SEQ ID NO: 25, HCDR2: SEQ ID NO: 46, and HCDR3: SEQ ID NO: 60;
27) HCDR1: SEQ ID NO: 26, HCDR2: SEQ ID NO: 46, and HCDR3: SEQ ID NO: 61;
28) HCDR1: SEQ ID NO: 27, HCDR2: SEQ ID NO: 46, and HCDR3: SEQ ID NO: 3;
29) HCDR1: SEQ ID NO: 28, HCDR2: SEQ ID NO: 52, and HCDR3: SEQ ID NO: 3;
30) HCDR1: SEQ ID NO: 29, HCDR2: SEQ ID NO: 46, and HCDR3: SEQ ID NO: 3;
31) HCDR1: SEQ ID NO: 25, HCDR2: SEQ ID NO: 52, and HCDR3: SEQ ID NO: 62;
32) HCDR1: SEQ ID NO: 9, HCDR2: SEQ ID NO: 50, and HCDR3: SEQ ID NO: 63;
33) HCDR1: SEQ ID NO: 30, HCDR2: SEQ ID NO: 46, and HCDR3: SEQ ID NO: 3;
34) HCDR1: SEQ ID NO: 31, HCDR2: SEQ ID NO: 46, and HCDR3: SEQ ID NO: 3;
35) HCDR1: SEQ ID NO: 32, HCDR2: SEQ ID NO: 53, and HCDR3: SEQ ID NO: 3;
36) HCDR1: SEQ ID NO: 33, HCDR2: SEQ ID NO: 52, and HCDR3: SEQ ID NO: 3;
37) HCDR1: SEQ ID NO: 34, HCDR2: SEQ ID NO: 46, and HCDR3: SEQ ID NO: 64;
38) HCDR1: SEQ ID NO: 35, HCDR2: SEQ ID NO: 46, and HCDR3: SEQ ID NO: 57;
39) HCDR1: SEQ ID NO: 9, HCDR2: SEQ ID NO: 54, and HCDR3: SEQ ID NO: 3;
40) HCDR1: SEQ ID NO: 9, HCDR2: SEQ ID NO: 46, and HCDR3: SEQ ID NO: 65;
41) HCDR1: SEQ ID NO: 36, HCDR2: SEQ ID NO: 46, and HCDR3: SEQ ID NO: 3;
42) HCDR1: SEQ ID NO: 37, HCDR2: SEQ ID NO: 52, and HCDR3: SEQ ID NO: 3;
43) HCDR1: SEQ ID NO: 9, HCDR2: SEQ ID NO: 46, and HCDR3: SEQ ID NO: 59;
44) HCDR1: SEQ ID NO: 38, HCDR2: SEQ ID NO: 54, and HCDR3: SEQ ID NO: 3;
45) HCDR1: SEQ ID NO: 39, HCDR2: SEQ ID NO: 46, and HCDR3: SEQ ID NO: 3;
46) HCDR1: SEQ ID NO: 40, HCDR2: SEQ ID NO: 46, and HCDR3: SEQ ID NO: 55;
47) HCDR1: SEQ ID NO: 41, HCDR2: SEQ ID NO: 46, and HCDR3: SEQ ID NO: 3;
48) HCDR1: SEQ ID NO: 9, HCDR2: SEQ ID NO: 46, and HCDR3: SEQ ID NO: 66;
49) HCDR1: SEQ ID NO: 9, HCDR2: SEQ ID NO: 46, and HCDR3: SEQ ID NO: 67.

In some embodiments, the isolated antigen binding protein includes a H-FR1, a C-terminus of the H-FR1 is directly or indirectly linked to an N-terminus of the HCDR1, and the H-FR1 includes an amino acid sequence as shown in SEQ ID NOs: 68, 144-148, 230, and/or 235.

In some embodiments, the isolated antigen binding protein includes a H-FR2, the H-FR2 is located between the HCDR1 and the HCDR2, and the H-FR2 includes an amino acid sequence as shown in SEQ ID NOs: 69, 149-151, and/or 231.

In some embodiments, the isolated antigen binding protein includes a H-FR3, the H-FR3 is located between the HCDR2 and the HCDR3, and the H-FR3 includes an amino acid sequence as shown in SEQ ID NOs: 70, 152-161, and/or 232.

In some embodiments, the isolated antigen binding protein includes a H-FR4, an N-terminus of the H-FR4 is directly or indirectly linked to a C-terminus of the HCDR3, and the H-FR4 includes an amino acid sequence as shown in SEQ ID NOs: 71, 162, and/or 233.

In some embodiments, the isolated antigen binding protein includes a H-FR1, a H-FR2, a H-FR3, and a H-FR4, and the H-FR1 includes an amino acid sequence as shown in SEQ ID NOs: 68, 144-148, 230, and/or 235; the H-FR2 includes an amino acid sequence as shown in SEQ ID NOs: 69, 149-151, and/or 231; the H-FR3 includes an amino acid sequence as shown in SEQ ID NOs: 70, 152-161, and/or 232; and the H-FR4 includes an amino acid sequence as shown in SEQ ID NOs: 71, 162, and/or 233.

In some embodiments, the isolated antigen binding protein includes a heavy chain variable region VH, and the VH includes an amino acid sequence as shown in SEQ ID NOs: 139 and/or 234.

In some embodiments, the isolated antigen binding protein includes a heavy chain variable region VH, and the VH includes an amino acid sequence as shown in SEQ ID NOs: 143 and/or 236.

In some embodiments, the VH of the isolated antigen binding protein includes an amino acid sequence as shown in any one of SEQ ID NOs: 72-120 and/or 163-227.

In some embodiments, the isolated antigen binding protein includes an antibody or an antigen binding fragment thereof.

In some embodiments, the isolated antigen binding protein includes a single-domain antibody or an antigen binding fragment thereof.

In some embodiments, the antigen binding fragment is selected from the group consisting of Fab, Fab', F(ab)2, Fv fragment, F(ab')2, scFv, di-scFv, VHH, and/or dAb.

In some embodiments, the antibody includes a chimeric antibody, a humanized antibody, and/or a fully human antibody.

In some embodiments, the isolated antigen binding protein includes an amino acid sequence as shown in any one of SEQ ID NOs: 72-120 and/or 163-227.

In some embodiments, the isolated antigen binding protein further includes an Fc region of an immunoglobulin.

In some embodiments, the VH of the antigen binding protein is directly or indirectly linked to the Fc region.

In some embodiments, a C-terminus of the VH of the antigen binding protein is directly or indirectly linked to an N-terminus of the Fc region.

In some embodiments, the VH of the antigen binding protein is fused with the Fc region in frame.

In some embodiments, the VH of the antigen binding protein is linked to the Fc region through a linker.

In some embodiments, the linker includes a peptide linker.

In some embodiments, the linker includes a flexible linker.

In some embodiments, the isolated antigen binding protein includes, sequentially from N-terminus to C-terminus, the VH of the antigen binding protein, the linker and the Fc region.

In some embodiments, the Fc region includes an Fc derived from IgG1 or an Fc derived from IgG4.

In some embodiments, the Fc region includes an amino acid sequence as shown in any one of SEQ ID NOs: 121-125.

In another aspect, the present application provides one or more polypeptides including the isolated antigen binding protein.

In another aspect, the present application provides one or more immunoconjugates including the isolated antigen binding protein or the polypeptide.

In another aspect, the present application provides one or more isolated nucleic acid molecules encoding the isolated antigen binding protein or the polypeptide.

In another aspect, the present application provides one or more vectors including the isolated nucleic acid molecule.

In another aspect, the present application provides one or more cells including and/or expressing the isolated antigen binding protein, the polypeptide, the immunoconjugate, the isolated nucleic acid molecule and/or the vector.

In another aspect, the present application provides a method for preparing the isolated antigen binding protein and/or the polypeptide, which includes culturing the cells under a condition allowing the expression of the isolated antigen binding protein and/or the polypeptide.

In another aspect, the present application provides one or more pharmaceutical compositions including the isolated antigen binding protein, the polypeptide, the immunoconjugate, the isolated nucleic acid molecule, the vector, the cell, and optionally a pharmaceutically acceptable adjuvant and/or excipient.

In another aspect, the present application provides a method for detecting the presence and/or content of PD-1, which includes:
administering the isolated antigen binding protein or the polypeptide.

In another aspect, the present application provides one or more kits including the isolated antigen binding protein or the polypeptide.

In some embodiments, the kit includes instructions for describing the method for detecting the presence and/or content of PD-1.

In another aspect, the present application provides a use of the isolated antigen binding protein or the polypeptide in the preparation of a kit used in a method for detecting the presence and/or content of PD-1.

In another aspect, the present application provides one or more uses of the isolated antigen binding protein and/or the polypeptide in the preparation of drugs for preventing and/or treating a disease or a disorder.

In another aspect, the present application provides one or more of the isolated antigen binding protein, the polypeptide, the immunoconjugate, the isolated nucleic acid molecule, the vector, the cell and/or the pharmaceutical composition, which is/are used for preventing, relieving, and/or treating a disease or a disorder.

In some embodiments, the diseases or disorder includes a tumor.

In some embodiments, the tumor includes a solid tumor.

In some embodiments, the tumor includes a blood tumor.

In some embodiments, the tumor includes a tumor associated with the expression of PD-L1.

In some embodiments, the tumor is selected from the group consisting of melanoma, lung cancer, head and neck squamous cell carcinoma, lymphoma, hepatocellular carcinoma, renal cell carcinoma, urothelial carcinoma, colorectal cancer, and breast cancer.

In another aspect, the present application provides a method for preventing and/or treating a disease or disorder, which includes administering to a subject in need an effective amount of the isolated antigen binding protein, the polypeptide, the immunoconjugate, the isolated nucleic acid molecule, the vector, and/or the cell.

In some embodiments, the diseases or disorder includes a tumor.

In some embodiments, the tumor includes a solid tumor.

In some embodiments, the tumor includes a blood tumor.

In some embodiments, the tumors include a tumor associated with the expression of PD-L1.

In some embodiments, the tumor is selected from the group consisting of melanoma, lung cancer, head and neck squamous cell carcinoma, lymphoma, hepatocellular carcinoma, renal cell carcinoma, urothelial carcinoma, colorectal cancer, and breast cancer.

In another aspect, the present application provides a method for inhibiting the interaction between PD-1 and PD-L1, which includes administering to a subject in need an effective amount of the isolated antigen binding protein, the polypeptide, the immunoconjugate, the isolated nucleic acid molecule, the vector, and/or the cell.

In another aspect, the present application provides the isolated antigen binding protein, the polypeptide, the immunoconjugate, the isolated nucleic acid molecule, the vector, the cell, and/or the pharmaceutical composition, which is used to inhibit the interaction between PD-1 and PD-L1.

In another aspect, the present application provides a use of the isolated antigen binding protein and/or the polypeptide in the preparation of drugs for inhibiting the interaction between PD-1 and PD-L1.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
Fig. 1 shows the SDS-PAGE protein expression results of the antigen binding protein of the present application.
Fig. 2 shows the binding activity of the antigen binding protein of the present application to recombinant human PD-1, recombinant monkey PD-1, and recombinant mouse PD-1.
Figs. 3A-3B show the affinity kinetics detection results of the MHPD1-AFc of the present application and pembrolizumab to the antigen protein.

### Detailed Description of the Embodiments

The implementation of the present application will be illustrated in the following specific examples, and other advantages and effects of the present application will be easily known by those familiar with this technology from the content disclosed in the specification.

### Definition of Terms

In the present application, the term "isolated" generally refers to being obtained artificially from the native state. If a certain "isolated" substance or ingredient appears in nature, it could be that its natural environment has changed, or the substance has been isolated from the natural environment, or both. For example, there is a certain non-isolated polynucleotide or polypeptide naturally occurring in a living animal, then the same polynucleotide or polypeptide of a high purity isolated from this natural state is called isolated. The term "isolated" does not exclude being mixed with artificial or synthetic substances, nor excluding the presence of other impure substances with no influences on the activity of the substance.

In the present application, the term "antigen binding protein" generally refers to a polypeptide molecule capable of specifically recognizing and/or neutralizing a particular antigen. In the present application, the term "antigen binding protein" may include "an antibody" or "an antigen binding fragment". For example, the antibody may include an immunoglobulin composed of at least two heavy (H) chains and two light (L) chains interconnected by disulfide bonds and may include any molecules containing the antigen binding portions thereof. The term "antibody" may include monoclonal antibodies, antibody fragments or antibody derivatives, including, but not limited to, camel-derived antibodies, fully human antibodies, chimeric antibodies, single-chain antibodies (e.g., scFv), and antigen binding antibody fragments (e.g., Fab, Fab' and (Fab)2 fragments). The term "antibody" may also include all the recombinant forms of the antibodies, such as antibodies expressed in prokaryotic cells, unglycosylated antibodies and any antigen binding antibody fragments and derivatives thereof described herein. In the present application, the "antibody" may include single-domain antibodies.

In the present application, the term "single-domain antibody" generally refers to a class of antibodies lacking the antibody light chain and having only the heavy chain variable region. It has been found in studies that there is a heavy chain antibody (hcAb) composed of only heavy chains but with complete functions in Bactrian camels, dromedaries, alpacas and llamas, and the variable domain of the hcAb (VHH for short) has a molecular weight of only 1/10 of that of conventional antibodies, which is the smallest molecular fragment with complete antibody functions currently available, called a single domain antibody (sdAb). Compared with other antibodies, the single-domain antibody has advantages of low immunogenicity, small molecule, and strong penetrability, thus having a broad application prospect in basic research, drug development, and disease treatment. For example, the single-domain antibody may be derived from alpacas. The single-domain antibody may be composed of a heavy chain variable region (VH). The term "heavy chain variable region" generally refers to an amino terminal domain of the heavy chain of the antigen binding fragment. The heavy chain variable region may be further divided into hypervariable regions called complementarity determining regions (CDRs) interspersed in more conservative regions called framework regions (FRs). Each heavy chain variable region may be composed of three CDRs and four FRs, which are arranged in the following order from the amino terminus to the carboxyl terminus: H-FR1, HCDR1, H-FR2, HCDR2, H-FR3, HCDR3, and H-FR4. The heavy chain variable region contains a binding domain interacting with the antigen (e.g., PD-1). The exact boundaries of CDRs have been defined differently depending on different systems. The system described by Kabat (Kabat et, al., Sequences of Proteins of Immunological Interest (National Institutes of Health, Bethesda, Md. (1987) and (1991)) not only provides a clear residue numbering system that can be applied to any variable regions of an antigen binding protein, but also provides precise residue boundaries for defining CDRs. These CDRs may be referred to as Kabat CDRs. Chothia and colleagues (Chothia and Lesk, J. Mol. Biol. 196:901-917 (1987) and Chothia et, al., Nature 342:877-883 (1989)) found that although there is considerable diversity at the amino acid sequence level, certain subportions within Kabat CDRs adopt nearly identical peptide backbone conformations. These subportions are named as L1, L2, and L3 or H1, H2, and H3, wherein "L" and "H" indicate light chain and heavy chain regions, respectively. These regions may be referred to as Chothia CDRs, which have boundaries overlapped with Kabat CDRs. Other boundaries for defining CDRs overlapped with Kabat CDRs have been described by Padlan (FASEB J.9:133-139 (1995)) and MacCallum (J Mol Biol 262 (5):732-45 (1996)). In addition, other definitions of CDR boundary may not strictly follow one of the above systems but will still overlap with Kabat CDRs, although they can be shortened or lengthened according to predictions or experimental findings that specific residues or groups of residues or even CDRs as a whole do not significantly affect antigen binding. In the present application, CDRs can be defined by IMGT numbering system. In the present application, the term "single-domain antibody"" can be used interchangeably with "nanobody" and "VHH". Although the scope claimed in the present application is the sequences as set forth based on the definition by the IMGT numbering system, amino acid sequences corresponding to other CDR definition rules also fall within the protection scope of the present application. Exemplary definition rules are shown in the table below.

| | IMGT | AbM | Kabat | Chothia |
|---|---|---|---|---|
| HCDR1 | 26-33 | 26-35 | 31-35 | 26-32 |
| HCDR2 | 51-58 | 50-59 | 50-66 | 52-57 |
| HCDR3 | 97-107 | 99-107 | 99-107 | 99-107 |

wherein, the amino acid range in the table refers to the amino acid numbering range starting from the N-terminus of the antigen binding protein of the present application. For example, "26-33" refers to an amino acid sequence from position 26 to position 33 starting from the N-terminus.

In the present application, the term "monoclonal antibody" generally refers to a population of substantially homogeneous antibodies, that is, various antibodies included in the population are the same except potential naturally occurring mutations present in a trace amount. The monoclonal antibodies are highly specific and directly target individual antigenic sites. For example, the monoclonal antibodies may be prepared by a hybridoma technology or produced in bacteria, animal or plant eukaryotic cells by using recombinant DNA methods. The monoclonal antibodies may also be obtained from a phage antibody library, by using a technology as described in, e.g., Clackson et, al., Nature, 352:624-628 (1991) and Marks et al., Mol. Biol., 222:581-597 (1991).

In the present application, the term "chimeric antibody" generally refers to such an antibody in which a part of each amino acid sequence of the heavy chain or the light chain is homogeneous to the corresponding amino acid sequence in an antibody derived from specific species or belongs to a particular class, while the residual segment of the chain is homogeneous to the corresponding sequence in another species. For example, the variable regions of the light chain and the heavy chain may be derived from the variable region of the antibody of an animal species (e.g., mice, rats, etc.), while the constant portion is homogeneous to the sequence of an antibody derived from another species (e.g., human). For example, to obtain a chimeric antibody, the variable region can be produced by using non-human B cells or hybridoma cells, while the constant region combined is derived from human. The variable region has an advantage of being easy to prepare and its specificity is not affected by the origin of the constant region with which it is combined. In addition, since the constant region of the chimeric antibody may be derived from human, the chimeric antibody is less likely to elicit an immune response when injected than using an antibody of which the constant region is not derived from human.

In the present application, the term "PD-1" or "PD1" generally refers to human programmed cell death receptor-1, a type I membrane protein of 288 amino acids, which is firstly described in 1992 (Ishida et al., EMBO J, 11 (1992), 3887-3895). PD-1 is a member of the expanded CD28/CTLA-4 T cell regulator family and has two ligands, PD-L1 (B7-H1, CD274) and PD-L2 (B7-DC, CD273). The structure of the protein includes an extracellular IgV domain, followed by a transmembrane region and an intracellular tail. The intracellular tail contains two phosphorylation sites located in the immunoreceptor tyrosine-based inhibitory motif and the immunoreceptor tyrosine-based switch motif, suggesting that PD-1 negatively regulates TCR signaling. This is consistent with the binding of phosphatases SHP-1 and SHP-2 to the cytoplasmic tail of PD-1 upon ligand binding. PD-1 is upregulated following T-cell receptor (TCR)-mediated activation and is observed on both activated and exhausted T cells (Agata et al., Int. Immunology 8 (1996),765-772). Although PD-1 exhibits a relatively broad expression pattern, its most significant role is likely as a co-inhibitory receptor on T cells (Chinai et al., Trends in Pharmacological Sciences 36 (2015), 587-595). Therefore, the focus is on blocking the interaction between PD-1 and its ligands to enhance T cell responses for the treatment of tumors. "PD-1 protein", "PD-1", PD1", PDCD1", "hPD-1" and "hPD-I" may be used interchangeably, and include human PD-1 isoforms, species homologues, functionally active fragments, and analogues that share at least one common epitope with PD-1. For example, the "functionally active fragment" may include fragments that retain at least one endogenous function of a naturally occurring protein (e.g., binding to the antigen binding protein of the present application). For example, the "functionally active fragment" may include a domain binding to the antigen binding protein of the present application. Exemplary amino acid sequences of human PD1 may be shown in UniProt Accession Number Q15116.

In the present application, the term "PD-L1" or "PDL1" generally refers to the programmed death receptor-ligand 1, also referred to as B7 homologue 1, B7-H1, cluster of differentiation 274, (3) 274 or CD274, which downregulates the activation of T cells and the secretion of cytokines upon binding to PD-1. "PD-L1" includes any natural PD-L1 derived from any vertebrates, including mammals, such as primates (e.g., human and cynomolgus macaques) and rodents (e.g., mice and rats). The term involves "full-length" unprocessed PD-L1 and any forms of PD-L1 produced from cell processing. PD-L1 may exist as transmembrane protein or as soluble protein. "PD-L1" includes complete PD-L1 and fragments thereof, further includes functional fragments, isoforms, species homologues, derivatives, analogues of PD-L1, as well as analogues that share at least one common epitope with PD-L1. For example, the "functionally active fragment" may include fragments that retain at least one endogenous function of a naturally occurring protein (e.g., binding to the antigen binding protein of the present application). For example, the "functionally active fragment" may include a domain binding to the antigen binding protein of the present application. Exemplary amino acid sequences of human PD1 may be found under NCBI Accession Number NP_001254653 or UniProt Accession Number Q9NZQ7.

In addition to the specific proteins and nucleotides mentioned herein, the present application may also include functional variants, derivatives, analogues, homologues, and fragments thereof.

The term "functional variant" refers to a polypeptide having substantially the same amino acid sequence or encoded by substantially the same nucleotide sequence as the naturally occurring sequence and capable of having one or more activities of the naturally occurring sequence. In the context of the present application, the variant of any given sequence refers to a sequence in which a particular sequence of residues (either amino acid or nucleotide residues) has been modified so that the polypeptide or polynucleotide retains substantially at least one endogenous function. The variant sequences can be obtained through the addition, deletion, substitution, modification, replacement and/or variation of at least one amino acid residue and/or nucleotide residue present in a naturally occurring protein and/or polynucleotide, as long as the original functional activity is maintained.

In the present application, the term "derivative" generally refers to a polypeptide or polynucleotide of the present application including any substitution, variation, modification, replacement, deletion and/or addition from/to one (or more) amino acid residues of the sequence, provided that the resulting polypeptide or polynucleotide substantially retains at least one of its endogenous functions.

In the present application, the term "analogue" generally, with respect to a polypeptide or polynucleotide, includes any mimetics of the polypeptide or polynucleotide, that is, chemical compounds having at least one endogenous function of the polypeptide or polynucleotide that the mimetics mimic.

In general, amino acids may be substituted, for example, at least 1 (e.g., 1, 2, 3, 4, 5, 6, 7, 8, 9, 10 or 20 or above) amino acids can be substituted, provided that the modified sequence substantially retains the desired activity or capability. Amino acid substitution may include the use of non-naturally occurring analogues.

In the present application, the term "homologue" generally refers to an amino acid sequence or a nucleotide sequence having a certain homology with a naturally occurring sequence. The term "homology" may be equivalent to the "identity" of sequences. Homologous sequences may include amino acid sequences that are at least 80%, 85%, 90%, 99.1%, 99.2%, 99.3%, 99.4%, 99.5%, 99.6%, 99.7%, 99.8% or 99.9% the same as the subject sequence. In general, homologues will contain the same active sites as the subject amino acid sequence, and the like. Homology may be considered on the basis of similarity (i.e., amino acid residues having similar chemical properties/functions), or homology can be expressed in terms of the sequence identity. In the present application, a sequence having a percentage of identity in either of the SEQ ID NOs of the mentioned amino acid sequence or nucleotide sequence refers to a sequence having the percentage of identity over the whole length of the mentioned SEQ ID NOs. In order to determine the sequence identity, alignment can be performed on sequences by a variety of ways known to those skilled in the art, for example, by using BLAST, BLAST-2, ALIGN, NEEDLE or Megalign (DNASTAR) software, etc. The persons skilled in the art are able to determine the proper parameters for alignment, including any algorithms required to achieve an optimal alignment in the full-length sequence being compared.

The protein or polypeptide used in the present application may also have deletion, insertion or substitution of amino acid residues, where the amino acid residues undergo silent changes and result in functionally equivalent proteins. Intentional amino acid substitutions can be made based on the similarity of the polarity, charge, solubility, hydrophobicity, hydrophilicity, and/or the amphoteric properties of the residues, as long as the endogenous function is retained. For example, negatively charged amino acids include aspartic acid and glutamic acid; positively charged amino acids include lysine and arginine; and amino acids containing uncharged polar head-groups with a similar hydrophilic value include asparagine, glutamine, serine, threonine and tyrosine.

In the present application, the term "tumor" generally refers to the neoplasm formed by the proliferation of local tissue cells under the action of various tumorigenic factors. For example, the tumors may include solid tumors. For example, the tumors may include blood tumors. For example, the tumors may include tumors associated with the expression of PD-L1. The term "tumors associated with the expression of PD-L1" generally refers to tumors formed by altered PD-L1 expression leading to disease progression or evasion of immune surveillance. For example, the "tumors associated with the expression of PD-L1" may be tumors formed by upregulation of expression level of PD-L1 leading to disease progression or evasion of immune surveillance. The tumors associated with the protein expression of PD-L1 may be PD-L1 positive tumors. In PD-L1 positive tumors, the protein expression level of PD-L1 on the surface of tumor cells or in the tumor microenvironment was approximately 1%, 5%, 10%, 15%, 20%, 25%, 30%, 35%, 40%, 50%, 60%, 70%, 80% or above higher than that in normal cells.

In the present application, the term "solid tumor" generally refers to a tangible mass that can be detected through clinical examinations such as X-ray, CT scan, B-ultrasonography, or palpation. For example, the solid tumor may be selected from the group consisting of melanoma, lung cancer, head and neck squamous cell carcinoma, hepatocellular carcinoma, renal cell carcinoma, urothelial carcinoma, colorectal cancer, and breast cancer.

In the present application, the term "blood tumor" generally refers to tumor that cannot be seen or touched by X-ray, CT scan, B-ultrasonography, or palpation. For example, the blood tumor may include leukemia. For example, the blood tumor may include lymphoma. For example, the blood tumor may include multiple myeloma.

In the present application, the term "immunoconjugate" generally refers to a conjugate formed by conjugating the other reagents (e.g., chemotherapeutic agents, radioactive elements, cell growth inhibitors and cytotoxic agents) with the isolated antigen binding protein (e.g., covalently linking), which can deliver the other reagents to target cells (e.g., tumor cells) by the specific binding of the isolated antigen binding protein to the antigens on the target cells. Then, the immunoconjugate is internalized, and finally goes to the interior of the target cells (e.g., into vesicles such as lysosome and the like), and at that time the linking molecule in the immunoconjugate can be cleaved to release the other reagents, thereby exerting its cytotoxic effect. Moreover, the antigen can also be secreted by the target cells and located in a gap outside the target cells.

In the present application, the term "subject" generally refers to human or non-human animals, including, but not limited to, cat, dog, horse, pig, cow, sheep, rabbit, mouse, rat, or monkey.

In the present application, the term "nucleic acid molecule" generally refers to isolated nucleotides, deoxyribonucleotides or ribonucleotides of any length, or analogues thereof isolated from their natural environment or synthesized artificially.

In the present application, the term "vector" generally refers to a nucleic acid molecule capable of transporting another nucleic acid to which it has been linked. The vector can transfer the inserted nucleic acid molecule into and/or between cells. The vector may include vectors mainly used for inserting DNA or RNA into cells, vectors mainly used for replicating DNA or RNA, and vectors mainly used for expression of DNA or RNA transcription and/or translation. The vector may be a polynucleotide that can be transcribed and translated into a polypeptide when introduced into a suitable cell. Generally, by culturing suitable cells comprising the vector, the vector can produce the desired expression product. In the present application, the vector may include lentiviral vector.

In the present application, the term "cell" generally refers to an individual cell, cell line, or cell culture that may include or has included a plasmid or vector comprising the nucleic acid molecule of the present application, or that can express the polypeptide of the present application or the antigen binding protein of the present application. The cells may include the progeny of a single cell. Due to natural, unintended, or intentional mutations, the progeny cells may not necessarily be completely the same as the original parent cells in terms of morphology or genome, as long as they can express the polypeptide or the antigen binding protein of the present application. The cells may be obtained by transfecting cells in vitro with the vector of the present application. The cells may be prokaryotic cells (e.g., *Escherichia coli*) or eukaryotic cells (e.g., yeast cells, e.g., COS cells, Chinese Hamster Ovary (CHO) cells, HeLa cells, HEK293 cells, COS-1 cells, NS0 cells, or myeloma cells). In some embodiments, the cells may be immune cells. For example, the immune cell may be selected from the group consisting of T cell, B cell, natural killer cell (NK cell), macrophage, NKT cell, monocyte, dendritic cell, granulocyte, lymphocyte, leukocyte, and/or peripheral blood mononuclear cell.

In the present application, the term "treatment" generally refers to: (i) preventing the occurrence of a disease, disorder, or condition in patients susceptible to but having not been diagnosed with the disease, disorder, and/or condition; (ii) inhibiting the disease, disorder, or condition, i.e., arresting its progression; and (iii) relieving the disease, disorder, or condition, i.e., causing regression of the disease, disorder, and/or condition and/or symptoms associated with the disease, disorder, and/or condition.

In the present application, the terms "polypeptide", "peptide", and "protein" can be used interchangeably and generally refer to a polymer with any length of amino acids. The polymer may be linear or branched, may include modified amino acid, and may be interrupted by non-amino acid. These terms also cover amino acid polymers that have been modified. These modifications may include disulfide bond formation, glycosylation, lipidation, acetylation, phosphorylation, or any other manipulation (e.g., binding to a labeling component). The term "amino acid" includes natural and/or non-natural or synthetic amino acid, including glycine and D and L optical isomer, as well as amino acid analogue and peptide mimetic.

In the present application, the terms "polynucleotide", "nucleotide", "nucleotide sequence", "nucleic acid", and "oligonucleotide" can be used interchangeably and generally refer to polymerized forms of nucleotides of any length, such as deoxyribonucleotide or ribonucleotide, or analogues thereof. Polynucleotide may have any three-dimensional structure and may perform any known or unknown function. Non-limiting examples of polynucleotides are as below: coding regions or non-coding regions of genes or gene fragments, multiple loci (one locus) defined according to linkage analysis, exon, intron, messenger RNA (mRNA), transfer RNA, ribosomal RNA, short interfering RNA (siRNA), short hairpin RNA (shRNA), micro-RNA (miRNA), ribozyme, cDNA, recombinant polynucleotides, branched polynucleotides, plasmids, vectors, isolated DNA of any sequence, isolated RNA of any sequence, nucleic acid probes, and primers. A polynucleotide may contain one or more modified nucleotides, such as methylated nucleotides and nucleotide analogues. If present, modification may be made to the nucleotide structure before or after the assembly of polymers. The sequence of nucleotide may be interrupted by non-nucleotide components. Polynucleotide may be further modified by conjugating with labelled components after polymerization.

In the present application, the term "K_{D}" (likewise, *"K_{D}"* or "KD") generally refers to "affinity constant" or "equilibrium dissociation constant" and refers to the value obtained at equilibrium in titration measurement, or by dividing the dissociation rate constant (k_{d}) by the association rate constant (kₐ). The association rate constant (kₐ), dissociation rate constant (k_{d}) and equilibrium dissociation constant (K_{D}) are used to indicate the binding affinity of the binding protein (e.g., the isolated antigen binding protein of the present application) to an antigen (e.g., PD-1 protein). Methods for determining the association and dissociation rate constants are well known in the art. The use of fluorescence-based techniques provides a high sensitivity and an ability of checking samples at equilibrium in physiological buffers. For example, the *K_{D}* value may be determined by Octet (Sartorius Co.), or by using other experimental approaches and instruments such as BIAcore (Biomolecular Interaction Analysis System, GE Healthcare Life Science). In addition, the *K_{D}* value may also be determined by using KinExA (Kinetic Exclusion Assay) of Sapidyne Instruments (Boise, Idaho) Company, or by using other surface plasmon resonance (SPR). In the present application, the *K_{D}* value may be determined by BIAcore.

In the present application, the term "and/or" should be understood as meaning any one or two of the alternatives.

In the present application, the term "include" generally means the inclusion of well-specified features, but not excluding other elements. In some cases, "include" also covers the instances where only the specified components are included. In some cases, "include" also covers the meaning of "consisting of".

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

In the present application, the term "include" generally refers to the meaning of comprising, encompassing, containing, or embracing. In some cases, it also means "is/are" or "be composed of... ".

### Detailed Description of the Invention

### Isolated antigen binding protein of the present application

In one aspect, the present application provides an isolated antigen binding protein, which may bind to PD-1 with an EC₅₀ of about 11 µg/mL or less (e.g., the EC₅₀ is not higher than about 11 µg/mL, not higher than about 10 µg/mL, not higher than about 9.5 µg/mL, not higher than about 9.0 µg/mL, not higher than about 8.5 µg/mL, not higher than about 8.0 µg/mL, not higher than about 7.5 µg/mL, not higher than about 7.0 µg/mL, not higher than 6.5 µg/mL, not higher than about 6.0 µg/mL, not higher than 5.5 µg/mL, not higher than about 5.0 µg/mL, not higher than about 4.5 µg/mL, not higher than about 4.0 µg/mL, not higher than about 3.5 µg/mL, not higher than about 3.0 µg/mL, not higher than about 2.5 µg/mL, not higher than about 2.0 µg/mL, not higher than about 1.5 µg/mL, not higher than about 1.0 µg/mL, not higher than about 0.5 µg/mL, not higher than about 0.4 µg/mL, not higher than about 0.3 µg/mL, not higher than about 0.2 µg/mL or not higher than about 0.1 µg/mL or below) in ELSA assay.

In the competitive ELISA assay, the isolated antigen binding protein of the present application may inhibit the binding of human PD-L1 to human PD-1 with an EC₅₀ of about 2 µg/mL or less (e.g., the EC₅₀ is not higher than about 1.9 µg/mL, not higher than about 1.8 µg/mL, not higher than about 1.7 µg/mL, not higher than about 1.5 µg/mL, not higher than about 1.3 µg/mL, not higher than about 1.1 µg/mL, not higher than about 1.0 µg/mL, not higher than about 0.9 µg/mL, not higher than 0.8 µg/mL, not higher than about 0.7 µg/mL, not higher than 0.6 µg/mL, not higher than about 0.5 µg/mL, not higher than about 0.4 µg/mL, not higher than about 0.3 µg/mL, not higher than about 0.2 µg/mL, or not higher than about 0.1 µg/mL or below).

In the present application, the isolated antigen binding protein may bind to the same or overlapping PD-1 epitope with a reference antibody. For example, the isolated antigen binding protein may compete with the reference antibody for binding to PD-1.

In the present application, the binding affinity of the isolated antigen binding protein for PD-1 may be substantially the same as the binding affinity of the reference antibody for PD-1. For example, the binding affinity may be determined by ELISA assay. For example, the binding affinity may be characterized by EC₅₀. For example, in the ELISA assay for determining the binding affinity, the EC₅₀ of the isolated antigen binding protein for PD-1 is approximately equal to or lower than the EC₅₀ of the reference antibody for PD-1.

In the present application, the reference antibody may include a heavy chain variable region VH, and the VH may include at least one, two, or three of HCDR1, HCDR2, and HCDR3.

In the present application, the exact boundaries of HCDRs may be defined differently depending on different systems. For example, the HCDRs of the present application may be defined by the Kabat system (see a system described by Kabat et, al., Sequences of Proteins of Immunological Interest (National Institutes of Health, Bethesda, Md. (1987) and (1991)), which not only provides a clear residue numbering system that can be applied to any variable regions of an antigen binding protein, but also provides precise residue boundaries for defining CDRs. These CDRs may be referred to as Kabat CDRs. For example, the HCDRs of the present application may also be defined by the Chothia system (see the studies by Chothia and colleagues (Chothia and Lesk, J. Mol. Biol. 196:901-917 (1987) and Chothia et, al., Nature 342:877-883 (1989))). Chothia and colleagues found that although there is considerable diversity at the amino acid sequence level, certain subportions within Kabat CDRs adopt nearly identical peptide backbone conformations. These subportions are named as L1, L2, and L3 or H1, H2, and H3, wherein "L" and "H" indicate light chain and heavy chain regions, respectively. These regions may be referred to as Chothia CDRs, which have boundaries overlapped with Kabat CDRs. Other boundaries for defining CDRs overlapped with Kabat CDRs have been described by Padlan (FASEB J.9:133-139 (1995)) and MacCallum (J Mol Biol 262 (5):732-45 (1996)). In addition, other definitions of CDR boundary may not strictly follow one of the above systems but will still overlap with Kabat CDRs, although they can be shortened or lengthened according to predictions or experimental findings that specific residues or groups of residues or even CDRs as a whole do not significantly affect antigen binding. In the present application, HCDRs can be determined by IMGT definition rules.

In the present application, the HCDR3 of the reference antibody may include an amino acid sequence as shown in SEQ ID NO: 138. For example, the sequence of the HCDR3 of the reference antibody may be determined according to IMGT definition rules.

AADX₄DX₆X₇GFDY (SEQ ID NO: 138), wherein, X₄ may be I or L, X₆ may be H, R or V, and X₇ may be A, D, G, H, L, N, R, S, T or V

In the present application, the HCDR3 of the reference antibody may include an amino acid sequence as shown in SEQ ID NO: 142. For example, the sequence of the HCDR3 of the reference antibody may be determined according to IMGT definition rules.

AADX₄DX₆X₇GFDY (SEQ ID NO: 142), wherein, X₄ may be I or L, X₆ may be H or R, and X₇ may be A, H or S.

In the present application, the HCDR3 of the reference antibody may include an amino acid sequence as shown in SEQ ID NOs: 3, 55, 56, 57, 58, 59, 60, 61, 62, 63, 64, 65, 66, and 67. For example, the sequence of the HCDR3 of the reference antibody may be determined according to IMGT definition rules.

In the present application, the HCDR2 of the reference antibody may include an amino acid sequence as shown in SEQ ID NO: 137. For example, the sequence of the HCDR2 of the reference antibody may be determined according to IMGT definition rules.

X₁GWX₄X₅X₆TX₈ (SEQ ID NO: 137), wherein, X₁ may be I or L, X₄ may be A, N, R or S, X₅ may be A, F, W or Y, X₆ may be A, G or S, and X₈ may be E, Q, S or T.

In the present application, the HCDR2 of the reference antibody may include an amino acid sequence as shown in SEQ ID NO: 141. For example, the sequence of the HCDR2 of the reference antibody may be determined according to IMGT definition rules.

IGWX₄X₅X₆TX₈ (SEQ ID NO: 141), wherein, X₄ may be A or N, X₅ may be F or Y, X₆ may be A or S, and X₈ may be E, Q or T.

In the present application, the HCDR2 of the reference antibody may include an amino acid sequence as shown in any one of SEQ ID NOs: 2, 42, 43, 44, 45, 46, 47, 48, 49, 50, 51, 52, 53, and 54. For example, the sequence of the HCDR2 of the reference antibody may be determined according to IMGT definition rules.

In the present application, the HCDR1 of the reference antibody may include an amino acid sequence as shown in SEQ ID NO: 136. For example, the sequence of the HCDR1 of the reference antibody may be determined according to IMGT definition rules.

X₁X₂X₃X₄X₅IYA (SEQ ID NO: 136), wherein, X₁ may be A, G, H, K, S or V, X₂ may be A, G, H, L, N, P, R, S, T or V, X₃ may be D, E, F, G, I, K, Q, R, S or V, X₄ may be D, G, H, K, L, P, Q, R, S, T, V or Y, and X₅ may be A, F, N, Q, R, SorT.

In the present application, the HCDR1 of the reference antibody may include an amino acid sequence as shown in SEQ ID NO: 140. For example, the sequence of the HCDR1 of the reference antibody may be determined according to IMGT definition rules.

X₁X₂X₃X₄X₅IYA (SEQ ID NO: 140), wherein, X₁ may be G, H or K, X₂ may be A, G, L, N, S or V, X₃ may be D, R or V, X₄ may be G, H, P, Q, R or T, and X₅ may be R, S or T.

In the present application, the HCDR1 of the reference antibody may include an amino acid sequence as shown in any one of SEQ ID NOs: 1, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 41. For example, the sequence of the HCDR1 of the reference antibody may be determined according to IMGT definition rules.

For example, the HCDR1 of the reference antibody may include an amino acid sequence as shown in SEQ ID NO: 138; the HCDR2 of the reference antibody may include an amino acid sequence as shown in SEQ ID NO: 137; and the HCDR3 of the reference antibody may include an amino acid sequence as shown in SEQ ID NO: 136.

For example, the HCDR1 of the reference antibody may include an amino acid sequence as shown in SEQ ID NO: 142; the HCDR2 of the reference antibody may include an amino acid sequence as shown in SEQ ID NO: 141; and the HCDR3 of the reference antibody may include an amino acid sequence as shown in SEQ ID NO: 140.

For example, the HCDR1 of the reference antibody may include an amino acid sequence as shown in SEQ ID NO: 1; the HCDR2 of the reference antibody may include an amino acid sequence as shown in SEQ ID NO: 2; and the HCDR3 of the reference antibody may include an amino acid sequence as shown in SEQ ID NO: 3. For example, the reference antibody may include a single-domain antibody MHPD1-A or an antibody having the same HCDRs 1-3 therewith.

For example, the HCDR1 of the reference antibody may include an amino acid sequence as shown in SEQ ID NO: 1; the HCDR2 of the reference antibody may include an amino acid sequence as shown in SEQ ID NO: 42; and the HCDR3 of the reference antibody may include an amino acid sequence as shown in SEQ ID NO: 55. For example, the reference antibody may include a single-domain antibody MHPD1-A-1 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 4; the HCDR2 of the reference antibody may include an amino acid sequence as shown in SEQ ID NO: 43; and the HCDR3 of the reference antibody may include an amino acid sequence as shown in SEQ ID NO: 3. For example, the reference antibody may include a single-domain antibody MHPD1-A-2 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 5; the HCDR2 of the reference antibody may include an amino acid sequence as shown in SEQ ID NO: 44; and the HCDR3 of the reference antibody may include an amino acid sequence as shown in SEQ ID NO: 3. For example, the reference antibody may include a single-domain antibody MHPD1-A-3 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 6; the HCDR2 of the reference antibody may include an amino acid sequence as shown in SEQ ID NO: 45; and the HCDR3 of the reference antibody may include an amino acid sequence as shown in SEQ ID NO: 3. For example, the reference antibody may include a single-domain antibody MHPD1-A-4 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 7; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-5 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 8; the HCDR2 of the reference antibody may include an amino acid sequence as shown in SEQ ID NO: 47; and the HCDR3 of the reference antibody may include an amino acid sequence as shown in SEQ ID NO: 3. For example, the reference antibody may include a single-domain antibody MHPD1-A-6 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-7 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 10; the HCDR2 of the reference antibody may include an amino acid sequence as shown in SEQ ID NO: 45; and the HCDR3 of the reference antibody may include an amino acid sequence as shown in SEQ ID NO: 3. For example, the reference antibody may include a single-domain antibody MHPD1-A-8 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 11; the HCDR2 of the reference antibody may include an amino acid sequence as shown in SEQ ID NO: 44; and the HCDR3 of the reference antibody may include an amino acid sequence as shown in SEQ ID NO: 3. For example, the reference antibody may include a single-domain antibody MHPD1-A-9 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 12; the HCDR2 of the reference antibody may include an amino acid sequence as shown in SEQ ID NO: 43; and the HCDR3 of the reference antibody may include an amino acid sequence as shown in SEQ ID NO: 3. For example, the reference antibody may include a single-domain antibody MHPD1-A-10 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 13; the HCDR2 of the reference antibody may include an amino acid sequence as shown in SEQ ID NO: 48; and the HCDR3 of the reference antibody may include an amino acid sequence as shown in SEQ ID NO: 3. For example, the reference antibody may include a single-domain antibody MHPD1-A-11 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 14; the HCDR2 of the reference antibody may include an amino acid sequence as shown in SEQ ID NO: 45; and the HCDR3 of the reference antibody may include an amino acid sequence as shown in SEQ ID NO: 3. For example, the reference antibody may include a single-domain antibody MHPD1-A-12 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 15; the HCDR2 of the reference antibody may include an amino acid sequence as shown in SEQ ID NO: 47; and the HCDR3 of the reference antibody may include an amino acid sequence as shown in SEQ ID NO: 3. For example, the reference antibody may include a single-domain antibody MHPD1-A-13 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 16; the HCDR2 of the reference antibody may include an amino acid sequence as shown in SEQ ID NO: 43; and the HCDR3 of the reference antibody may include an amino acid sequence as shown in SEQ ID NO: 3. For example, the reference antibody may include a single-domain antibody MHPD1-A-14 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 17; the HCDR2 of the reference antibody may include an amino acid sequence as shown in SEQ ID NO: 49; and the HCDR3 of the reference antibody may include an amino acid sequence as shown in SEQ ID NO: 3. For example, the reference antibody may include a single-domain antibody MHPD1-A-15 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 18; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-16 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 19; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-17 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 20; the HCDR2 of the reference antibody may include an amino acid sequence as shown in SEQ ID NO: 50; and the HCDR3 of the reference antibody may include an amino acid sequence as shown in SEQ ID NO: 56. For example, the reference antibody may include a single-domain antibody MHPD1-A-18 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 21; the HCDR2 of the reference antibody may include an amino acid sequence as shown in SEQ ID NO: 46; and the HCDR3 of the reference antibody may include an amino acid sequence as shown in SEQ ID NO: 57. For example, the reference antibody may include a single-domain antibody MHPD1-A-19 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 22; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-20 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 51; and the HCDR3 of the reference antibody may include an amino acid sequence as shown in SEQ ID NO: 3. For example, the reference antibody may include a single-domain antibody MHPD1-A-21 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 46; and the HCDR3 of the reference antibody may include an amino acid sequence as shown in SEQ ID NO: 58. For example, the reference antibody may include a single-domain antibody MHPD1-A-22 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 23; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-23 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 24; the HCDR2 of the reference antibody may include an amino acid sequence as shown in SEQ ID NO: 46; and the HCDR3 of the reference antibody may include an amino acid sequence as shown in SEQ ID NO: 59. For example, the reference antibody may include a single-domain antibody MHPD1-A-24 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 25; the HCDR2 of the reference antibody may include an amino acid sequence as shown in SEQ ID NO: 46; and the HCDR3 of the reference antibody may include an amino acid sequence as shown in SEQ ID NO: 60. For example, the reference antibody may include a single-domain antibody MHPD1-A-25 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 26; the HCDR2 of the reference antibody may include an amino acid sequence as shown in SEQ ID NO: 46; and the HCDR3 of the reference antibody may include an amino acid sequence as shown in SEQ ID NO: 61. For example, the reference antibody may include a single-domain antibody MHPD1-A-26 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 27; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-27 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 28; the HCDR2 of the reference antibody may include an amino acid sequence as shown in SEQ ID NO: 52; and the HCDR3 of the reference antibody may include an amino acid sequence as shown in SEQ ID NO: 3. For example, the reference antibody may include a single-domain antibody MHPD1-A-28 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 29; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-29 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 25; the HCDR2 of the reference antibody may include an amino acid sequence as shown in SEQ ID NO: 52; and the HCDR3 of the reference antibody may include an amino acid sequence as shown in SEQ ID NO: 62. For example, the reference antibody may include a single-domain antibody MHPD1-A-30 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 50; and the HCDR3 of the reference antibody may include an amino acid sequence as shown in SEQ ID NO: 63. For example, the reference antibody may include a single-domain antibody MHPD1-A-31 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 30; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-32 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 31; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-33 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 32; the HCDR2 of the reference antibody may include an amino acid sequence as shown in SEQ ID NO: 53; and the HCDR3 of the reference antibody may include an amino acid sequence as shown in SEQ ID NO: 3. For example, the reference antibody may include a single-domain antibody MHPD1-A-35 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 33; the HCDR2 of the reference antibody may include an amino acid sequence as shown in SEQ ID NO: 52; and the HCDR3 of the reference antibody may include an amino acid sequence as shown in SEQ ID NO: 3. For example, the reference antibody may include a single-domain antibody MHPD1-A-36 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 34; the HCDR2 of the reference antibody may include an amino acid sequence as shown in SEQ ID NO: 46; and the HCDR3 of the reference antibody may include an amino acid sequence as shown in SEQ ID NO: 64. For example, the reference antibody may include a single-domain antibody MHPD1-A-37 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 35; the HCDR2 of the reference antibody may include an amino acid sequence as shown in SEQ ID NO: 46; and the HCDR3 of the reference antibody may include an amino acid sequence as shown in SEQ ID NO: 57. For example, the reference antibody may include a single-domain antibody MHPD1-A-38 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 54; and the HCDR3 of the reference antibody may include an amino acid sequence as shown in SEQ ID NO: 3. For example, the reference antibody may include a single-domain antibody MHPD1-A-39 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 46; and the HCDR3 of the reference antibody may include an amino acid sequence as shown in SEQ ID NO: 65. For example, the reference antibody may include a single-domain antibody MHPD1-A-40 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 36; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-41 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 37; the HCDR2 of the reference antibody may include an amino acid sequence as shown in SEQ ID NO: 52; and the HCDR3 of the reference antibody may include an amino acid sequence as shown in SEQ ID NO: 3. For example, the reference antibody may include a single-domain antibody MHPD1-A-42 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 46; and the HCDR3 of the reference antibody may include an amino acid sequence as shown in SEQ ID NO: 59. For example, the reference antibody may include a single-domain antibody MHPD1-A-43 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 38; the HCDR2 of the reference antibody may include an amino acid sequence as shown in SEQ ID NO: 54; and the HCDR3 of the reference antibody may include an amino acid sequence as shown in SEQ ID NO: 3. For example, the reference antibody may include a single-domain antibody MHPD1-A-44 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 39; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-45 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 40; the HCDR2 of the reference antibody may include an amino acid sequence as shown in SEQ ID NO: 46; and the HCDR3 of the reference antibody may include an amino acid sequence as shown in SEQ ID NO: 55. For example, the reference antibody may include a single-domain antibody MHPD1-A-46 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 41; the HCDR2 of the reference antibody may include an amino acid sequence as shown in SEQ ID NO: 46; and the HCDR3 of the reference antibody may include an amino acid sequence as shown in SEQ ID NO: 3. For example, the reference antibody may include a single-domain antibody MHPD1-A-47 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 46; and the HCDR3 of the reference antibody may include an amino acid sequence as shown in SEQ ID NO: 66. For example, the reference antibody may include a single-domain antibody MHPD1-A-48 or an antigen binding protein (e.g., a single-domain antibody) having the same HCDR3 therewith (e.g., having the same HCDRs 1-3 therewith).

For example, the HCDR1 of the reference antibody may include an amino acid sequence as shown in SEQ ID NO: 9; the HCDR2 of the reference antibody may include an amino acid sequence as shown in SEQ ID NO: 46; and the HCDR3 of the reference antibody may include an amino acid sequence as shown in SEQ ID NO: 67. For example, the reference antibody may include a single-domain antibody MHPD1-A-49 or an antigen binding protein (e.g., a single-domain antibody) having the same HCDR3 therewith (e.g., having the same HCDRs 1-3 therewith).

For example, the VH of the reference antibody may include framework regions H-FR1, H-FR2, H-FR3, and H-FR4.

In the present application, the H-FR1 may include an amino acid sequence as shown in SEQ ID NOs: 68, 144-148, 230, and/or 235.

In the present application, the H-FR2 may include an amino acid sequence as shown in SEQ ID NOs: 69, 149-151, and/or 231.

In the present application, the H-FR3 may include an amino acid sequence as shown in SEQ ID NOs: 70, 152-161, and/or 232.

In the present application, the H-FR4 may include an amino acid sequence as shown in SEQ ID NOs: 71, 162, and/or 233.

In the present application, the H-FR1 of the reference antibody may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; and the H-FR4 may include an amino acid sequence as shown in SEQ ID NO: 71. For example, the reference antibody may include a single-domain antibody MHPD1-A or an antibody having the same H-FRs 1-4 therewith.

In the present application, the reference antibody may include a heavy chain variable region, and the heavy chain variable region may include an amino acid sequence as shown in SEQ ID NO: 139.

QVQLVESGGGSVQVGGSLTLSCAASX₂₆X₂₇X₂₈X₂₉X₃₀IYAMGWFRQAPGKEHEFVA GX₅₁GWX₅₄X₅₅X₅₆TX₅₈YYADSVKGRFGISRDNTKNTVALLMNSLKPEDTAIYYCAADX ₁₀₀DX₁₀₂X₁₀₃GFDYWGQGTQVTVSS (SEQ ID NO: 139), wherein, X₂₆ may be A, G, H, K, S or V, X₂₇ may be A, G, H, L, N, P, R, S, T or V, X₂₈ may be D, E, F, G, I, K, Q, R, S or V, X₂₉ may be D, G, H, K, L, P, Q, R, S, T, V or Y, X₃₀ may be A, F, N, Q, R, S or T, X₅₁ may be I or L, X₅₄ may be A, N, R or S, X₅₅ may be A, F, W or Y, X₅₆ may be A, G or S, X₅₈ may be E, Q, SorT, X₁₀₀ may be I or L, X₁₀₂ may be H, R or V, and X₁₀₃ may be A, D, G, H, L, N, R, S, T or V.

In the present application, the reference antibody may include a heavy chain variable region, and the heavy chain variable region may include an amino acid sequence as shown in SEQ ID NO: 143.

QVQLVESGGGSVQVGGSLTLSCAASX₂₆X₂₇X₂₈X₂₉X₃₀IYAMGWFRQAPGKEHEFV AGIGWX₅₄X₅₅X₅₆TX₅₈YYADSVKGRFGISRDNTKNTVALLMNSLKPEDTAIYYCAADX_{1 00}DX₁₀₂X₁₀₃GFDYWGQGTQVTVSS (SEQ ID NO: 143), wherein, X₂₆ may be G, H or K, X₂₇ may be A, G, L, N, S or V, X₂₈ may be D, R or V, X₂₉ may be G, H, P, Q, R or T, X₃₀ may be R, S or T, X₅₄ may be A or N, X₅₅ may be F or Y, X₅₆ may be A or S, X₅₈ may be E, Q or T, X₁₀₀ may be I or L, X₁₀₂ may be H or R, and X₁₀₃ may be A, H or S.

In the present application, the heavy chain variable region of the reference antibody may include an amino acid sequence as shown in any one of SEQ ID NOs: 72-120 and/or 163-227.

In the present application, the HCDR3 of the reference antibody may include an amino acid sequence as shown in SEQ ID NO: 128.

In the present application, the HCDR2 of the reference antibody may include an amino acid sequence as shown in SEQ ID NO: 127.

In the present application, the HCDR1 of the reference antibody may include an amino acid sequence as shown in SEQ ID NO: 126.

For example, the HCDR1 of the reference antibody may include an amino acid sequence as shown in SEQ ID NO: 126; the HCDR2 of the reference antibody may include an amino acid sequence as shown in SEQ ID NO: 127; and the HCDR3 of the reference antibody may include an amino acid sequence as shown in SEQ ID NO: 128. For example, the reference antibody may include pembrolizumab or an antibody having the same HCDRs 1-3 therewith.

In the present application, the reference antibody may include a light chain variable region VL, and the VL may include LCDR1, LCDR2, and LCDR3.

In the present application, the LCDR3 of the reference antibody may include an amino acid sequence as shown in SEQ ID NO: 131.

In the present application, the LCDR2 of the reference antibody may include an amino acid sequence as shown in SEQ ID NO: 130.

In the present application, the LCDR1 of the reference antibody may include an amino acid sequence as shown in SEQ ID NO: 129.

For example, the LCDR1 of the reference antibody may include an amino acid sequence as shown in SEQ ID NO: 129; the LCDR2 of the reference antibody may include an amino acid sequence as shown in SEQ ID NO: 130; and the LCDR3 of the reference antibody may include an amino acid sequence as shown in SEQ ID NO: 131. For example, the reference antibody may include pembrolizumab or an antibody having the same LCDRs 1-3 therewith.

For example, the HCDR1 of the reference antibody may include an amino acid sequence as shown in SEQ ID NO: 126; the HCDR2 of the reference antibody may include an amino acid sequence as shown in SEQ ID NO: 127; the HCDR3 of the reference antibody may include an amino acid sequence as shown in SEQ ID NO: 128; the LCDR1 of the reference antibody may include an amino acid sequence as shown in SEQ ID NO: 129; the LCDR2 of the reference antibody may include an amino acid sequence as shown in SEQ ID NO: 130; the LCDR3 of the reference antibody may include an amino acid sequence as shown in SEQ ID NO: 131. For example, the reference antibody may include pembrolizumab or an antigen binding protein having the same HCDRs 1-3 and LCDRs 1-3 therewith.

For example, the VH of the reference antibody may include an amino acid sequence as shown in SEQ ID NO: 132. For example, the VL of the reference antibody may include an amino acid sequence as shown in SEQ ID NO: 133. For example, the reference antibody may include and pembrolizumab or an antigen binding protein having the same VH and VL therewith.

In the present application, the reference antibody may include a heavy chain and a light chain. For example, the heavy chain of the reference antibody may include an amino acid sequence as shown in SEQ ID NO: 134. For example, the light chain of the reference antibody may include an amino acid sequence as shown in SEQ ID NO: 135. For example, the reference antibody may include and pembrolizumab or an antigen binding protein having the same heavy chain and light chain therewith.

In the present application, the antigen binding protein may include a heavy chain variable region VH, and the VH may include at least one, two, or three of HCDR1, HCDR2, and HCDR3.

In the present application, the HCDR3 of the antigen binding protein may include an amino acid sequence as shown in SEQ ID NO: 138. For example, the sequence of the HCDR3 of the antigen binding protein may be determined according to IMGT definition rules.

AADX₄DX₆X₇GFDY (SEQ ID NO: 138), wherein, X₄ may be I or L, X₆ may be H, R or V, and X₇ may be A, D, G, H, L, N, R, S, T or V

In the present application, the HCDR3 of the antigen binding protein may include an amino acid sequence as shown in SEQ ID NO: 142. For example, the sequence of the HCDR3 of the antigen binding protein may be determined according to IMGT definition rules.

AADX₄DX₆X₇GFDY (SEQ ID NO: 142), wherein, X₄ may be I or L, X₆ may be H or R, and X7 may be A, H or S.

In the present application, the HCDR3 of the antigen binding protein may include an amino acid sequence as shown in any one of SEQ ID NOs: 3, 55, 56, 57, 58, 59, 60, 61, 62, 63, 64, 65, 66, and 67. For example, the sequence of the HCDR3 of the antigen binding protein may be determined according to IMGT definition rules.

In the present application, the HCDR2 of the antigen binding protein may include an amino acid sequence as shown in SEQ ID NO: 137. For example, the sequence of the HCDR2 of the antigen binding protein may be determined according to IMGT definition rules.

X₁GWX₄X₅X₆TX₈ (SEQ ID NO: 137), wherein, X₁ may be I or L, X₄ may be A, N, R or S, X₅ may be A, F, W or Y, X₆ may be A, G or S, and X₈ may be E, Q, S or T.

In the present application, the HCDR2 of the antigen binding protein may include an amino acid sequence as shown in SEQ ID NO: 141. For example, the sequence of the HCDR2 of the antigen binding protein may be determined according to IMGT definition rules.

IGWX₄X₅X₆TX₈ (SEQ ID NO: 141), wherein, X₄ may be A or N, X₅ may be F or Y, X₆ may be A or S, and X₈ may be E, Q or T.

In the present application, the HCDR2 of the antigen binding protein may include an amino acid sequence as shown in any one of SEQ ID NOs: 2, 42, 43, 44, 45, 46, 47, 48, 49, 50, 51, 52, 53, and 54. For example, the sequence of the HCDR2 of the antigen binding protein may be determined according to IMGT definition rules.

In the present application, the HCDR1 of the antigen binding protein may include an amino acid sequence as shown in SEQ ID NO: 136. For example, the sequence of the HCDR1 of the antigen binding protein may be determined according to IMGT definition rules.

X₁X₂X₃X₄X₅IYA (SEQ ID NO: 136), wherein, X₁ may be A, G, H, K, S or V, X₂ may be A, G, H, L, N, P, R, S, T or V, X₃ may be D, E, F, G, I, K, Q, R, S or V, X₄ may be D, G, H, K, L, P, Q, R, S, T, V or Y, and X₅ may be A, F, N, Q, R, S or T.

In the present application, the HCDR1 of the antigen binding protein may include an amino acid sequence as shown in SEQ ID NO: 140. For example, the sequence of the HCDR1 of the antigen binding protein may be determined according to IMGT definition rules.

X₁X₂X₃X₄X₅IYA (SEQ ID NO: 140), wherein, X₁ may be G, H or K, X₂ may be A, G, L, N, S or V, X₃ may be D, R or V, X₄ may be G, H, P, Q, R or T, and X₅ may be R, S or T.

In the present application, the HCDR1 of the antigen binding protein may include an amino acid sequence as shown in any one of SEQ ID NOs: 1, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 41. For example, the sequence of the HCDR1 of the antigen binding protein may be determined according to IMGT definition rules.

For example, the HCDR1 of the antigen binding protein may include an amino acid sequence as shown in SEQ ID NO: 138; the HCDR2 of the antigen binding protein may include an amino acid sequence as shown in SEQ ID NO: 137; and the HCDR3 of the antigen binding protein may include an amino acid sequence as shown in SEQ ID NO: 136.

For example, the HCDR1 of the antigen binding protein may include an amino acid sequence as shown in SEQ ID NO: 142; the HCDR2 of the antigen binding protein may include an amino acid sequence as shown in SEQ ID NO: 141; and the HCDR3 of the antigen binding protein may include an amino acid sequence as shown in SEQ ID NO: 140.

For example, the HCDR1 of the antigen binding protein may include an amino acid sequence as shown in SEQ ID NO: 1; the HCDR2 of the antigen binding protein may include an amino acid sequence as shown in SEQ ID NO: 2; and the HCDR3 of the antigen binding protein may include an amino acid sequence as shown in SEQ ID NO: 3. For example, the antigen binding protein may include a single-domain antibody MHPD1-A or an antibody having the same HCDRs 1-3 therewith.

For example, the HCDR1 of the antigen binding protein may include an amino acid sequence as shown in SEQ ID NO: 1; the HCDR2 of the antigen binding protein may include an amino acid sequence as shown in SEQ ID NO: 42; and the HCDR3 of the antigen binding protein may include an amino acid sequence as shown in SEQ ID NO: 55. For example, the antigen binding protein may include a single-domain antibody MHPD1-A-1 or an antibody having the same HCDRs 1-3 therewith.

For example, the HCDR1 of the antigen binding protein may include an amino acid sequence as shown in SEQ ID NO: 4; the HCDR2 of the antigen binding protein may include an amino acid sequence as shown in SEQ ID NO: 43; and the HCDR3 of the antigen binding protein may include an amino acid sequence as shown in SEQ ID NO: 3. For example, the antigen binding protein may include a single-domain antibody MHPD1-A-2 or an antibody having the same HCDRs 1-3 therewith.

For example, the HCDR1 of the antigen binding protein may include an amino acid sequence as shown in SEQ ID NO: 5; the HCDR2 of the antigen binding protein may include an amino acid sequence as shown in SEQ ID NO: 44; and the HCDR3 of the antigen binding protein may include an amino acid sequence as shown in SEQ ID NO: 3. For example, the antigen binding protein may include a single-domain antibody MHPD1-A-3 or an antibody having the same HCDRs 1-3 therewith.

For example, the HCDR1 of the antigen binding protein may include an amino acid sequence as shown in SEQ ID NO: 6; the HCDR2 of the antigen binding protein may include an amino acid sequence as shown in SEQ ID NO: 45; and the HCDR3 of the antigen binding protein may include an amino acid sequence as shown in SEQ ID NO: 3. For example, the antigen binding protein may include a single-domain antibody MHPD1-A-4 or an antibody having the same HCDRs 1-3 therewith.

For example, the HCDR1 of the antigen binding protein may include an amino acid sequence as shown in SEQ ID NO: 7; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-5 or an antibody having the same HCDRs 1-3 therewith.

For example, the HCDR1 of the antigen binding protein may include an amino acid sequence as shown in SEQ ID NO: 8; the HCDR2 of the antigen binding protein may include an amino acid sequence as shown in SEQ ID NO: 47; and the HCDR3 of the antigen binding protein may include an amino acid sequence as shown in SEQ ID NO: 3. For example, the antigen binding protein may include a single-domain antibody MHPD1-A-6 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-7 or an antibody having the same HCDRs 1-3 therewith.

For example, the HCDR1 of the antigen binding protein may include an amino acid sequence as shown in SEQ ID NO: 10; the HCDR2 of the antigen binding protein may include an amino acid sequence as shown in SEQ ID NO: 45; and the HCDR3 of the antigen binding protein may include an amino acid sequence as shown in SEQ ID NO: 3. For example, the antigen binding protein may include a single-domain antibody MHPD1-A-8 or an antibody having the same HCDRs 1-3 therewith.

For example, the HCDR1 of the antigen binding protein may include an amino acid sequence as shown in SEQ ID NO: 11; the HCDR2 of the antigen binding protein may include an amino acid sequence as shown in SEQ ID NO: 44; and the HCDR3 of the antigen binding protein may include an amino acid sequence as shown in SEQ ID NO: 3. For example, the antigen binding protein may include a single-domain antibody MHPD1-A-9 or an antibody having the same HCDRs 1-3 therewith.

For example, the HCDR1 of the antigen binding protein may include an amino acid sequence as shown in SEQ ID NO: 12; the HCDR2 of the antigen binding protein may include an amino acid sequence as shown in SEQ ID NO: 43; and the HCDR3 of the antigen binding protein may include an amino acid sequence as shown in SEQ ID NO: 3. For example, the antigen binding protein may include a single-domain antibody MHPD1-A-10 or an antibody having the same HCDRs 1-3 therewith.

For example, the HCDR1 of the antigen binding protein may include an amino acid sequence as shown in SEQ ID NO: 13; the HCDR2 of the antigen binding protein may include an amino acid sequence as shown in SEQ ID NO: 48; and the HCDR3 of the antigen binding protein may include an amino acid sequence as shown in SEQ ID NO: 3. For example, the antigen binding protein may include a single-domain antibody MHPD1-A-11 or an antibody having the same HCDRs 1-3 therewith.

For example, the HCDR1 of the antigen binding protein may include an amino acid sequence as shown in SEQ ID NO: 14; the HCDR2 of the antigen binding protein may include an amino acid sequence as shown in SEQ ID NO: 45; and the HCDR3 of the antigen binding protein may include an amino acid sequence as shown in SEQ ID NO: 3. For example, the antigen binding protein may include a single-domain antibody MHPD1-A-12 or an antibody having the same HCDRs 1-3 therewith.

For example, the HCDR1 of the antigen binding protein may include an amino acid sequence as shown in SEQ ID NO: 15; the HCDR2 of the antigen binding protein may include an amino acid sequence as shown in SEQ ID NO: 47; and the HCDR3 of the antigen binding protein may include an amino acid sequence as shown in SEQ ID NO: 3. For example, the antigen binding protein may include a single-domain antibody MHPD1-A-13 or an antibody having the same HCDRs 1-3 therewith.

For example, the HCDR1 of the antigen binding protein may include an amino acid sequence as shown in SEQ ID NO: 16; the HCDR2 of the antigen binding protein may include an amino acid sequence as shown in SEQ ID NO: 43; and the HCDR3 of the antigen binding protein may include an amino acid sequence as shown in SEQ ID NO: 3. For example, the antigen binding protein may include a single-domain antibody MHPD1-A-14 or an antibody having the same HCDRs 1-3 therewith.

For example, the HCDR1 of the antigen binding protein may include an amino acid sequence as shown in SEQ ID NO: 17; the HCDR2 of the antigen binding protein may include an amino acid sequence as shown in SEQ ID NO: 49; and the HCDR3 of the antigen binding protein may include an amino acid sequence as shown in SEQ ID NO: 3. For example, the antigen binding protein may include a single-domain antibody MHPD1-A-15 or an antibody having the same HCDRs 1-3 therewith.

For example, the HCDR1 of the antigen binding protein may include an amino acid sequence as shown in SEQ ID NO: 18; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-16 or an antibody having the same HCDRs 1-3 therewith.

For example, the HCDR1 of the antigen binding protein may include an amino acid sequence as shown in SEQ ID NO: 19; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-17 or an antibody having the same HCDRs 1-3 therewith.

For example, the HCDR1 of the antigen binding protein may include an amino acid sequence as shown in SEQ ID NO: 20; the HCDR2 of the antigen binding protein may include an amino acid sequence as shown in SEQ ID NO: 50; and the HCDR3 of the antigen binding protein may include an amino acid sequence as shown in SEQ ID NO: 56. For example, the antigen binding protein may include a single-domain antibody MHPD1-A-18 or an antibody having the same HCDRs 1-3 therewith.

For example, the HCDR1 of the antigen binding protein may include an amino acid sequence as shown in SEQ ID NO: 21; the HCDR2 of the antigen binding protein may include an amino acid sequence as shown in SEQ ID NO: 46; and the HCDR3 of the antigen binding protein may include an amino acid sequence as shown in SEQ ID NO: 57. For example, the antigen binding protein may include a single-domain antibody MHPD1-A-19 or an antibody having the same HCDRs 1-3 therewith.

For example, the HCDR1 of the antigen binding protein may include an amino acid sequence as shown in SEQ ID NO: 22; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-20 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 51; and the HCDR3 of the antigen binding protein may include an amino acid sequence as shown in SEQ ID NO: 3. For example, the antigen binding protein may include a single-domain antibody MHPD1-A-21 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 46; and the HCDR3 of the antigen binding protein may include an amino acid sequence as shown in SEQ ID NO: 58. For example, the antigen binding protein may include a single-domain antibody MHPD1-A-22 or an antibody having the same HCDRs 1-3 therewith.

For example, the HCDR1 of the antigen binding protein may include an amino acid sequence as shown in SEQ ID NO: 23; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-23 or an antibody having the same HCDRs 1-3 therewith.

For example, the HCDR1 of the antigen binding protein may include an amino acid sequence as shown in SEQ ID NO: 24; the HCDR2 of the antigen binding protein may include an amino acid sequence as shown in SEQ ID NO: 46; and the HCDR3 of the antigen binding protein may include an amino acid sequence as shown in SEQ ID NO: 59. For example, the antigen binding protein may include a single-domain antibody MHPD1-A-24 or an antibody having the same HCDRs 1-3 therewith.

For example, the HCDR1 of the antigen binding protein may include an amino acid sequence as shown in SEQ ID NO: 25; the HCDR2 of the antigen binding protein may include an amino acid sequence as shown in SEQ ID NO: 46; and the HCDR3 of the antigen binding protein may include an amino acid sequence as shown in SEQ ID NO: 60. For example, the antigen binding protein may include a single-domain antibody MHPD1-A-25 or an antibody having the same HCDRs 1-3 therewith.

For example, the HCDR1 of the antigen binding protein may include an amino acid sequence as shown in SEQ ID NO: 26; the HCDR2 of the antigen binding protein may include an amino acid sequence as shown in SEQ ID NO: 46; and the HCDR3 of the antigen binding protein may include an amino acid sequence as shown in SEQ ID NO: 61. For example, the antigen binding protein may include a single-domain antibody MHPD1-A-26 or an antibody having the same HCDRs 1-3 therewith.

For example, the HCDR1 of the antigen binding protein may include an amino acid sequence as shown in SEQ ID NO: 27; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-27 or an antibody having the same HCDRs 1-3 therewith.

For example, the HCDR1 of the antigen binding protein may include an amino acid sequence as shown in SEQ ID NO: 28; the HCDR2 of the antigen binding protein may include an amino acid sequence as shown in SEQ ID NO: 52; and the HCDR3 of the antigen binding protein may include an amino acid sequence as shown in SEQ ID NO: 3. For example, the antigen binding protein may include a single-domain antibody MHPD1-A-28 or an antibody having the same HCDRs 1-3 therewith.

For example, the HCDR1 of the antigen binding protein may include an amino acid sequence as shown in SEQ ID NO: 29; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-29 or an antibody having the same HCDRs 1-3 therewith.

For example, the HCDR1 of the antigen binding protein may include an amino acid sequence as shown in SEQ ID NO: 25; the HCDR2 of the antigen binding protein may include an amino acid sequence as shown in SEQ ID NO: 52; and the HCDR3 of the antigen binding protein may include an amino acid sequence as shown in SEQ ID NO: 62. For example, the antigen binding protein may include a single-domain antibody MHPD1-A-30 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 50; and the HCDR3 of the antigen binding protein may include an amino acid sequence as shown in SEQ ID NO: 63. For example, the antigen binding protein may include a single-domain antibody MHPD1-A-31 or an antibody having the same HCDRs 1-3 therewith.

For example, the HCDR1 of the antigen binding protein may include an amino acid sequence as shown in SEQ ID NO: 30; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-32 or an antibody having the same HCDRs 1-3 therewith.

For example, the HCDR1 of the antigen binding protein may include an amino acid sequence as shown in SEQ ID NO: 31; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-33 or an antibody having the same HCDRs 1-3 therewith.

For example, the HCDR1 of the antigen binding protein may include an amino acid sequence as shown in SEQ ID NO: 32; the HCDR2 of the antigen binding protein may include an amino acid sequence as shown in SEQ ID NO: 53; and the HCDR3 of the antigen binding protein may include an amino acid sequence as shown in SEQ ID NO: 3. For example, the antigen binding protein may include a single-domain antibody MHPD1-A-35 or an antibody having the same HCDRs 1-3 therewith.

For example, the HCDR1 of the antigen binding protein may include an amino acid sequence as shown in SEQ ID NO: 33; the HCDR2 of the antigen binding protein may include an amino acid sequence as shown in SEQ ID NO: 52; and the HCDR3 of the antigen binding protein may include an amino acid sequence as shown in SEQ ID NO: 3. For example, the antigen binding protein may include a single-domain antibody MHPD1-A-36 or an antibody having the same HCDRs 1-3 therewith.

For example, the HCDR1 of the antigen binding protein may include an amino acid sequence as shown in SEQ ID NO: 34; the HCDR2 of the antigen binding protein may include an amino acid sequence as shown in SEQ ID NO: 46; and the HCDR3 of the antigen binding protein may include an amino acid sequence as shown in SEQ ID NO: 64. For example, the antigen binding protein may include a single-domain antibody MHPD1-A-37 or an antibody having the same HCDRs 1-3 therewith.

For example, the HCDR1 of the antigen binding protein may include an amino acid sequence as shown in SEQ ID NO: 35; the HCDR2 of the antigen binding protein may include an amino acid sequence as shown in SEQ ID NO: 46; and the HCDR3 of the antigen binding protein may include an amino acid sequence as shown in SEQ ID NO: 57. For example, the antigen binding protein may include a single-domain antibody MHPD1-A-38 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 54; and the HCDR3 of the antigen binding protein may include an amino acid sequence as shown in SEQ ID NO: 3. For example, the antigen binding protein may include a single-domain antibody MHPD1-A-39 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 46; and the HCDR3 of the antigen binding protein may include an amino acid sequence as shown in SEQ ID NO: 65. For example, the antigen binding protein may include a single-domain antibody MHPD1-A-40 or an antibody having the same HCDRs 1-3 therewith.

For example, the HCDR1 of the antigen binding protein may include an amino acid sequence as shown in SEQ ID NO: 36; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-41 or an antibody having the same HCDRs 1-3 therewith.

For example, the HCDR1 of the antigen binding protein may include an amino acid sequence as shown in SEQ ID NO: 37; the HCDR2 of the antigen binding protein may include an amino acid sequence as shown in SEQ ID NO: 52; and the HCDR3 of the antigen binding protein may include an amino acid sequence as shown in SEQ ID NO: 3. For example, the antigen binding protein may include a single-domain antibody MHPD1-A-42 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 46; and the HCDR3 of the antigen binding protein may include an amino acid sequence as shown in SEQ ID NO: 59. For example, the antigen binding protein may include a single-domain antibody MHPD1-A-43 or an antibody having the same HCDRs 1-3 therewith.

For example, the HCDR1 of the antigen binding protein may include an amino acid sequence as shown in SEQ ID NO: 38; the HCDR2 of the antigen binding protein may include an amino acid sequence as shown in SEQ ID NO: 54; and the HCDR3 of the antigen binding protein may include an amino acid sequence as shown in SEQ ID NO: 3. For example, the antigen binding protein may include a single-domain antibody MHPD1-A-44 or an antibody having the same HCDRs 1-3 therewith.

For example, the HCDR1 of the antigen binding protein may include an amino acid sequence as shown in SEQ ID NO: 39; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-45 or an antibody having the same HCDRs 1-3 therewith.

For example, the HCDR1 of the antigen binding protein may include an amino acid sequence as shown in SEQ ID NO: 40; the HCDR2 of the antigen binding protein may include an amino acid sequence as shown in SEQ ID NO: 46; and the HCDR3 of the antigen binding protein may include an amino acid sequence as shown in SEQ ID NO: 55. For example, the antigen binding protein may include a single-domain antibody MHPD1-A-46 or an antibody having the same HCDRs 1-3 therewith.

For example, the HCDR1 of the antigen binding protein may include an amino acid sequence as shown in SEQ ID NO: 41; the HCDR2 of the antigen binding protein may include an amino acid sequence as shown in SEQ ID NO: 46; and the HCDR3 of the antigen binding protein may include an amino acid sequence as shown in SEQ ID NO: 3. For example, the antigen binding protein may include a single-domain antibody MHPD1-A-47 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 46; and the HCDR3 of the antigen binding protein may include an amino acid sequence as shown in SEQ ID NO: 66. For example, the antigen binding protein may include a single-domain antibody MHPD1-A-48 or an antibody having the same HCDRs 1-3 therewith.

For example, the HCDR1 of the antigen binding protein may include an amino acid sequence as shown in SEQ ID NO: 9; the HCDR2 of the antigen binding protein may include an amino acid sequence as shown in SEQ ID NO: 46; and the HCDR3 of the antigen binding protein may include an amino acid sequence as shown in SEQ ID NO: 67. For example, the antigen binding protein may include a single-domain antibody MHPD1-A-49 or an antibody having the same HCDRs 1-3 therewith.

For example, the VH of the antigen binding protein may include framework regions H-FR1, H-FR2, H-FR3, and H-FR4.

In the present application, the H-FR1 may include an amino acid sequence as shown in SEQ ID NO: 230 and/or 235. In the present application, the H-FR2 may include an amino acid sequence as shown in SEQ ID NO: 231. In the present application, the H-FR3 may include an amino acid sequence as shown in SEQ ID NO: 232. In the present application, the H-FR4 may include an amino acid sequence as shown in SEQ ID NO: 233.

In the present application, the H-FR1 may include an amino acid sequence as shown in SEQ ID NOs: 68, 144-148, and/or 230.

In the present application, the H-FR2 may include an amino acid sequence as shown in SEQ ID NOs: 69 and/or 149-151.

In the present application, the H-FR3 may include an amino acid sequence as shown in SEQ ID NOs: 70 and/or 152-161.

In the present application, the H-FR4 may include an amino acid sequence as shown in SEQ ID NOs: 71 and/or 162.

In the present application, the H-FR1 of the antigen binding protein may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; and the H-FR4 may include an amino acid sequence as shown in SEQ ID NO: 71. For example, the antigen binding protein may include a single-domain antibody MHPD1-A or an antibody having the same H-FRs 1-4 therewith.

In the present application, the antigen binding protein may include a heavy chain variable region, and the heavy chain variable region may include an amino acid sequence as shown in SEQ ID NO: 234.

QVQLVESGGGX₁VQX₂GGSLX₃LSCAX₄SX₅X₆X₇X₈X₉IYAX₁₀X₁₁WFRQAPGKX₁₂X ₁₃EFVAGX₁₄GWX₁₅X₁₆X₁₇TX₁₈YYADSVKGRFX₁₉ISRDNX₂₀KNTX₂₁X₂₂LX₂₃MNSLX₂₄X_{2 5}EDTAX₂₆YYCAADX₂₇DX₂₈X₂₉GFDYWGQGTX₃₀VTVSS (SEQ ID NO: 234), wherein, X₁ may be L or S, X₂ may be P or V, X₃ may be R or T, X₄ may be A or T, X₅ may be A, G, H, K, S, or V, X₆ may be A, G, H, L, N, P, R, S, T, or V, X₇ may be D, E, F, G, I, K, Q, R, S, or V, X₈ may be D, G, H, K, L, P, Q, R, S, T, V, or Y, X₉ may be A, F, N, Q, R, S, or T, X₁₀ may be A or M, Xu may be G or M, X₁₂ may be E or G, X₁₃ may be H or L, X₁₄ may be I or L, X₁₅ may be A, N, R, or S, X₁₆ may be A, F, W, or Y, X₁₇ may be A, G, or S, X₁₈ may be E, Q, S, or T, X₁₉ may be G or T, X₂₀ may be S or T, X₂₁ may be L or V, X₂₂ may be A or Y, X₂₃ may be L or Q, X₂₄ may be K or R, X₂₅ may be A or P, X₂₆ may be I or V, X₂₇ may be I or L, X₂₈ may be H, R, or V, X₂₉ may be A, D, G, H, L, N, R, S, T, or V, and X₃₀ may be L or Q.

In the present application, the antigen binding protein may include a heavy chain variable region, and the heavy chain variable region may include an amino acid sequence as shown in SEQ ID NO: 236.

QVQLVESGGGLVQPGGSLRLSCAX₁SX₂X₃X₄X₅X₆IYAMGWFRQAPGKGLEFVAGI GWX₇X₈X₉TX₁₀YYADSVKGRFTISRDNTKNTLYLQMNSLRAEDTAVYYCAADX₁₁DX₁₂ X₁₃GFDYWGQGTLVTVSS (SEQ ID NO: 236), wherein, X₁ may be A or T, X₂ may be G, H, or K, X₃ may be A, G, L, N, S, or V, X₄ may be D, R, or V, X₅ may be G, H, P, Q, R, or T, X₆ may be R, S, or T, X₇ may be A or N, Xs may be F or Y, X₉ may be A or S, X₁₀ may be E, Q, or T, X₁₁ may be I or L, X₁₂ may be H or R, and X₁₃ may be A, H, or S.

In the present application, the antigen binding protein may include a heavy chain variable region, and the heavy chain variable region may include an amino acid sequence as shown in SEQ ID NO: 139.

QVQLVESGGGSVQVGGSLTLSCAASX₂₆X₂₇X₂₈X₂₉X₃₀IYAMGWFRQAPGKEHEFVA GX₅₁GWX₅₄X₅₅X₅₆TX₅₈YYADSVKGRFGISRDNTKNTVALLMNSLKPEDTAIYYCAADX ₁₀₀DX₁₀₂X₁₀₃GFDYWGQGTQVTVSS (SEQ ID NO: 139), wherein, X₂₆ may be A, G, H, K, S or V, X₂₇ may be A, G, H, L, N, P, R, S, T or V, X₂₈ may be D, E, F, G, I, K, Q, R, S or V, X₂₉ may be D, G, H, K, L, P, Q, R, S, T, V or Y, X₃₀ may be A, F, N, Q, R, S or T, X₅₁ may be I or L, X₅₄ may be A, N, R or S, X₅₅ may be A, F, W or Y, X₅₆ may be A, G or S, X₅₈ may be E, Q, SorT, X₁₀₀ may be I or L, X₁₀₂ may be H, R or V, and X₁₀₃ may be A, D, G, H, L, N, R, S, T or V.

In the present application, the antigen binding protein may include a heavy chain variable region, and the heavy chain variable region may include an amino acid sequence as shown in SEQ ID NO: 143.

QVQLVESGGGSVQVGGSLTLSCAASX₂₆X₂₇X₂₈X₂₉X₃₀IYAMGWFRQAPGKEHEFV AGIGWX₅₄X₅₅X₅₆TX₅₈YYADSVKGRFGISRDNTKNTVALLMNSLKPEDTAIYYCAADX_{1 00}DX₁₀₂X₁₀₃GFDYWGQGTQVTVSS (SEQ ID NO: 143), wherein, X₂₆ may be G, H or K, X₂₇ may be A, G, L, N, S or V, X₂₈ may be D, R or V, X₂₉ may be G, H, P, Q, R or T, X₃₀ may be R, S or T, X₅₄ may be A or N, X₅₅ may be F or Y, X₅₆ may be A or S, X₅₈ may be E, Q or T, X₁₀₀ may be I or L, X₁₀₂ may be H or R, and X₁₀₃ may be A, H or S.

In the present application, the heavy chain variable region may include an amino acid sequence as shown in any one of SEQ ID NOs: 72-120 and/or 163-227.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 1; the HCDR2 may include an amino acid sequence as shown in SEQ ID NO: 2; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A or an antigen binding protein having the same HCDRs 1-3 and H-FRs 1-4 therewith. For example, the heavy chain variable region of the antigen binding protein may include an amino acid sequence as shown in SEQ ID NO: 72. For example, the antigen binding protein may include a single-domain antibody MHPD1-A or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 1; the HCDR2 may include an amino acid sequence as shown in SEQ ID NO: 42; the HCDR3 may include an amino acid sequence as shown in SEQ ID NO: 55; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-1 or an antigen binding protein having the same HCDRs 1-3 and H-FRs 1-4 therewith. For example, the heavy chain variable region of the antigen binding protein may include an amino acid sequence as shown in SEQ ID NO: 73. For example, the antigen binding protein may include a single-domain antibody MHPD1-A-1 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-1.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 4; the HCDR2 may include an amino acid sequence as shown in SEQ ID NO: 43; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-2 or an antigen binding protein having the same HCDRs 1-3 and H-FRs 1-4 therewith. For example, the heavy chain variable region of the antigen binding protein may include an amino acid sequence as shown in SEQ ID NO: 74. For example, the antigen binding protein may include a single-domain antibody MHPD1-A-2 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-2.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 5; the HCDR2 may include an amino acid sequence as shown in SEQ ID NO: 44; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-3 or an antigen binding protein having the same HCDRs 1-3 and H-FRs 1-4 therewith. For example, the heavy chain variable region of the antigen binding protein may include an amino acid sequence as shown in SEQ ID NO: 75. For example, the antigen binding protein may include a single-domain antibody MHPD1-A-3 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-3.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 6; the HCDR2 may include an amino acid sequence as shown in SEQ ID NO: 45; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-4 or an antigen binding protein having the same HCDRs 1-3 and H-FRs 1-4 therewith. For example, the heavy chain variable region of the antigen binding protein may include an amino acid sequence as shown in SEQ ID NO: 76. For example, the antigen binding protein may include a single-domain antibody MHPD1-A-4 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-4.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 6; the HCDR2 may include an amino acid sequence as shown in SEQ ID NO: 45; the HCDR3 may include an amino acid sequence as shown in SEQ ID NO: 3; the H-FR1 may include an amino acid sequence as shown in SEQ ID NO: 147; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-4 H or an antigen binding protein having the same HCDRs 1-3 and H-FRs 1-4 therewith. For example, the heavy chain variable region of the antigen binding protein may include an amino acid sequence as shown in SEQ ID NO: 182. For example, the antigen binding protein may include a single-domain antibody MHPD1-A-4 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-4 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 7; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-5 or an antigen binding protein having the same HCDRs 1-3 and H-FRs 1-4 therewith. For example, the heavy chain variable region of the antigen binding protein may include an amino acid sequence as shown in SEQ ID NO: 77. For example, the antigen binding protein may include a single-domain antibody MHPD1-A-5 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 8; the HCDR2 may include an amino acid sequence as shown in SEQ ID NO: 47; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-6 or an antigen binding protein having the same HCDRs 1-3 and H-FRs 1-4 therewith. For example, the heavy chain variable region of the antigen binding protein may include an amino acid sequence as shown in SEQ ID NO: 78. For example, the antigen binding protein may include a single-domain antibody MHPD1-A-6 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-6.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-7 or an antigen binding protein having the same HCDRs 1-3 and H-FRs 1-4 therewith. For example, the heavy chain variable region of the antigen binding protein may include an amino acid sequence as shown in SEQ ID NO: 79. For example, the antigen binding protein may include a single-domain antibody MHPD1-A-7 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-7.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-7 H or an antigen binding protein having the same HCDRs 1-3 and H-FRs 1-4 therewith. For example, the heavy chain variable region of the antigen binding protein may include an amino acid sequence as shown in SEQ ID NO: 185. For example, the antigen binding protein may include a single-domain antibody MHPD1-A-7 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-7 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 10; the HCDR2 may include an amino acid sequence as shown in SEQ ID NO: 45; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-8 or an antigen binding protein having the same HCDRs 1-3 and H-FRs 1-4 therewith. For example, the heavy chain variable region of the antigen binding protein may include an amino acid sequence as shown in SEQ ID NO: 80. For example, the antigen binding protein may include a single-domain antibody MHPD1-A-8 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-8.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 10; the HCDR2 may include an amino acid sequence as shown in SEQ ID NO: 45; the HCDR3 may include an amino acid sequence as shown in SEQ ID NO: 3; the H-FR1 may include an amino acid sequence as shown in SEQ ID NO: 147; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-8 H or an antigen binding protein having the same HCDRs 1-3 and H-FRs 1-4 therewith. For example, the heavy chain variable region of the antigen binding protein may include an amino acid sequence as shown in SEQ ID NO: 186. For example, the antigen binding protein may include a single-domain antibody MHPD1-A-8 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-8 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 11; the HCDR2 may include an amino acid sequence as shown in SEQ ID NO: 44; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-9 or an antigen binding protein having the same HCDRs 1-3 and H-FRs 1-4 therewith. For example, the heavy chain variable region of the antigen binding protein may include an amino acid sequence as shown in SEQ ID NO: 81. For example, the antigen binding protein may include a single-domain antibody MHPD1-A-9 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-9.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 12; the HCDR2 may include an amino acid sequence as shown in SEQ ID NO: 43; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-10 or an antigen binding protein having the same HCDRs 1-3 and H-FRs 1-4 therewith. For example, the heavy chain variable region of the antigen binding protein may include an amino acid sequence as shown in SEQ ID NO: 82. For example, the antigen binding protein may include a single-domain antibody MHPD1-A-10 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-10.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 13; the HCDR2 may include an amino acid sequence as shown in SEQ ID NO: 48; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-11 or an antigen binding protein having the same HCDRs 1-3 and H-FRs 1-4 therewith. For example, the heavy chain variable region of the antigen binding protein may include an amino acid sequence as shown in SEQ ID NO: 83. For example, the antigen binding protein may include a single-domain antibody MHPD1-A-11 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-11.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 14; the HCDR2 may include an amino acid sequence as shown in SEQ ID NO: 45; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-12 or an antigen binding protein having the same HCDRs 1-3 and H-FRs 1-4 therewith. For example, the heavy chain variable region of the antigen binding protein may include an amino acid sequence as shown in SEQ ID NO: 84. For example, the antigen binding protein may include a single-domain antibody MHPD1-A-12 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-12.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 15; the HCDR2 may include an amino acid sequence as shown in SEQ ID NO: 47; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-13 or an antigen binding protein having the same HCDRs 1-3 and H-FRs 1-4 therewith. For example, the heavy chain variable region of the antigen binding protein may include an amino acid sequence as shown in SEQ ID NO: 85. For example, the antigen binding protein may include a single-domain antibody MHPD1-A-13 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-13.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 15; the HCDR2 may include an amino acid sequence as shown in SEQ ID NO: 47; the HCDR3 may include an amino acid sequence as shown in SEQ ID NO: 3; the H-FR1 may include an amino acid sequence as shown in SEQ ID NO: 147; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-13 H or an antigen binding protein having the same HCDRs 1-3 and H-FRs 1-4 therewith. For example, the heavy chain variable region of the antigen binding protein may include an amino acid sequence as shown in SEQ ID NO: 191. For example, the antigen binding protein may include a single-domain antibody MHPD1-A-13 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-13 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 16; the HCDR2 may include an amino acid sequence as shown in SEQ ID NO: 43; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-14 or an antigen binding protein having the same HCDRs 1-3 and H-FRs 1-4 therewith. For example, the heavy chain variable region of the antigen binding protein may include an amino acid sequence as shown in SEQ ID NO: 86. For example, the antigen binding protein may include a single-domain antibody MHPD1-A-14 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-14.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 17; the HCDR2 may include an amino acid sequence as shown in SEQ ID NO: 49; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-15 or an antigen binding protein having the same HCDRs 1-3 and H-FRs 1-4 therewith. For example, the heavy chain variable region of the antigen binding protein may include an amino acid sequence as shown in SEQ ID NO: 87. For example, the antigen binding protein may include a single-domain antibody MHPD1-A-15 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-15.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 17; the HCDR2 may include an amino acid sequence as shown in SEQ ID NO: 49; the HCDR3 may include an amino acid sequence as shown in SEQ ID NO: 3; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-15 H or an antigen binding protein having the same HCDRs 1-3 and H-FRs 1-4 therewith. For example, the heavy chain variable region of the antigen binding protein may include an amino acid sequence as shown in SEQ ID NO: 193. For example, the antigen binding protein may include a single-domain antibody MHPD1-A-15 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-15 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 18; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-16 or an antigen binding protein having the same HCDRs 1-3 and H-FRs 1-4 therewith. For example, the heavy chain variable region of the antigen binding protein may include an amino acid sequence as shown in SEQ ID NO: 88. For example, the antigen binding protein may include a single-domain antibody MHPD1-A-16 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-16.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 19; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-17 or an antigen binding protein having the same HCDRs 1-3 and H-FRs 1-4 therewith. For example, the heavy chain variable region of the antigen binding protein may include an amino acid sequence as shown in SEQ ID NO: 89. For example, the antigen binding protein may include a single-domain antibody MHPD1-A-17 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-17.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 20; the HCDR2 may include an amino acid sequence as shown in SEQ ID NO: 50; the HCDR3 may include an amino acid sequence as shown in SEQ ID NO: 56; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-18 or an antigen binding protein having the same HCDRs 1-3 and H-FRs 1-4 therewith. For example, the heavy chain variable region of the antigen binding protein may include an amino acid sequence as shown in SEQ ID NO: 90. For example, the antigen binding protein may include a single-domain antibody MHPD1-A-18 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-18.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 21; the HCDR2 may include an amino acid sequence as shown in SEQ ID NO: 46; the HCDR3 may include an amino acid sequence as shown in SEQ ID NO: 57; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-19 or an antigen binding protein having the same HCDRs 1-3 and H-FRs 1-4 therewith. For example, the heavy chain variable region of the antigen binding protein may include an amino acid sequence as shown in SEQ ID NO: 91. For example, the antigen binding protein may include a single-domain antibody MHPD1-A-19 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-19.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 22; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-20 or an antigen binding protein having the same HCDRs 1-3 and H-FRs 1-4 therewith. For example, the heavy chain variable region of the antigen binding protein may include an amino acid sequence as shown in SEQ ID NO: 92. For example, the antigen binding protein may include a single-domain antibody MHPD1-A-20 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-20.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 51; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-21 or an antigen binding protein having the same HCDRs 1-3 and H-FRs 1-4 therewith. For example, the heavy chain variable region of the antigen binding protein may include an amino acid sequence as shown in SEQ ID NO: 93. For example, the antigen binding protein may include a single-domain antibody MHPD1-A-21 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-21.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 46; the HCDR3 may include an amino acid sequence as shown in SEQ ID NO: 58; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-22 or an antigen binding protein having the same HCDRs 1-3 and H-FRs 1-4 therewith. For example, the heavy chain variable region of the antigen binding protein may include an amino acid sequence as shown in SEQ ID NO: 94. For example, the antigen binding protein may include a single-domain antibody MHPD1-A-22 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-22.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 23; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-23 or an antigen binding protein having the same HCDRs 1-3 and H-FRs 1-4 therewith. For example, the heavy chain variable region of the antigen binding protein may include an amino acid sequence as shown in SEQ ID NO: 95. For example, the antigen binding protein may include a single-domain antibody MHPD1-A-23 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-23.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 24; the HCDR2 may include an amino acid sequence as shown in SEQ ID NO: 46; the HCDR3 may include an amino acid sequence as shown in SEQ ID NO: 59; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-24 or an antigen binding protein having the same HCDRs 1-3 and H-FRs 1-4 therewith. For example, the heavy chain variable region of the antigen binding protein may include an amino acid sequence as shown in SEQ ID NO: 96. For example, the antigen binding protein may include a single-domain antibody MHPD1-A-24 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-24.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 24; the HCDR2 may include an amino acid sequence as shown in SEQ ID NO: 46; the HCDR3 may include an amino acid sequence as shown in SEQ ID NO: 59; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-24 H or an antigen binding protein having the same HCDRs 1-3 and H-FRs 1-4 therewith. For example, the heavy chain variable region of the antigen binding protein may include an amino acid sequence as shown in SEQ ID NO: 202. For example, the antigen binding protein may include a single-domain antibody MHPD1-A-24 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-24 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 25; the HCDR2 may include an amino acid sequence as shown in SEQ ID NO: 46; the HCDR3 may include an amino acid sequence as shown in SEQ ID NO: 60; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-25 or an antigen binding protein having the same HCDRs 1-3 and H-FRs 1-4 therewith. For example, the heavy chain variable region of the antigen binding protein may include an amino acid sequence as shown in SEQ ID NO: 97. For example, the antigen binding protein may include a single-domain antibody MHPD1-A-25 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-25.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 25; the HCDR2 may include an amino acid sequence as shown in SEQ ID NO: 46; the HCDR3 may include an amino acid sequence as shown in SEQ ID NO: 60; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-25 H or an antigen binding protein having the same HCDRs 1-3 and H-FRs 1-4 therewith. For example, the heavy chain variable region of the antigen binding protein may include an amino acid sequence as shown in SEQ ID NO: 203. For example, the antigen binding protein may include a single-domain antibody MHPD1-A-25 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-25 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 26; the HCDR2 may include an amino acid sequence as shown in SEQ ID NO: 46; the HCDR3 may include an amino acid sequence as shown in SEQ ID NO: 61; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-26 or an antigen binding protein having the same HCDRs 1-3 and H-FRs 1-4 therewith. For example, the heavy chain variable region of the antigen binding protein may include an amino acid sequence as shown in SEQ ID NO: 98. For example, the antigen binding protein may include a single-domain antibody MHPD1-A-26 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-26.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 27; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-27 or an antigen binding protein having the same HCDRs 1-3 and H-FRs 1-4 therewith. For example, the heavy chain variable region of the antigen binding protein may include an amino acid sequence as shown in SEQ ID NO: 99. For example, the antigen binding protein may include a single-domain antibody MHPD1-A-27 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-27.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 28; the HCDR2 may include an amino acid sequence as shown in SEQ ID NO: 52; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-28 or an antigen binding protein having the same HCDRs 1-3 and H-FRs 1-4 therewith. For example, the heavy chain variable region of the antigen binding protein may include an amino acid sequence as shown in SEQ ID NO: 100. For example, the antigen binding protein may include a single-domain antibody MHPD1-A-28 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-28.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 29; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-29 or an antigen binding protein having the same HCDRs 1-3 and H-FRs 1-4 therewith. For example, the heavy chain variable region of the antigen binding protein may include an amino acid sequence as shown in SEQ ID NO: 101. For example, the antigen binding protein may include a single-domain antibody MHPD1-A-29 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-29.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 25; the HCDR2 may include an amino acid sequence as shown in SEQ ID NO: 52; the HCDR3 may include an amino acid sequence as shown in SEQ ID NO: 62; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-30 or an antigen binding protein having the same HCDRs 1-3 and H-FRs 1-4 therewith. For example, the heavy chain variable region of the antigen binding protein may include an amino acid sequence as shown in SEQ ID NO: 102. For example, the antigen binding protein may include a single-domain antibody MHPD1-A-30 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-30.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 25; the HCDR2 may include an amino acid sequence as shown in SEQ ID NO: 52; the HCDR3 may include an amino acid sequence as shown in SEQ ID NO: 62; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-30 H or an antigen binding protein having the same HCDRs 1-3 and H-FRs 1-4 therewith. For example, the heavy chain variable region of the antigen binding protein may include an amino acid sequence as shown in SEQ ID NO: 208. For example, the antigen binding protein may include a single-domain antibody MHPD1-A-30 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-30 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 50; the HCDR3 may include an amino acid sequence as shown in SEQ ID NO: 63; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-31 or an antigen binding protein having the same HCDRs 1-3 and H-FRs 1-4 therewith. For example, the heavy chain variable region of the antigen binding protein may include an amino acid sequence as shown in SEQ ID NO: 103. For example, the antigen binding protein may include a single-domain antibody MHPD1-A-31 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-31.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 30; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-32 or an antigen binding protein having the same HCDRs 1-3 and H-FRs 1-4 therewith. For example, the heavy chain variable region of the antigen binding protein may include an amino acid sequence as shown in SEQ ID NO: 104. For example, the antigen binding protein may include a single-domain antibody MHPD1-A-32 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-32.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 30; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-32 H or an antigen binding protein having the same HCDRs 1-3 and H-FRs 1-4 therewith. For example, the heavy chain variable region of the antigen binding protein may include an amino acid sequence as shown in SEQ ID NO: 210. For example, the antigen binding protein may include a single-domain antibody MHPD1-A-32 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-32 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 31; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-33 or an antigen binding protein having the same HCDRs 1-3 and H-FRs 1-4 therewith. For example, the heavy chain variable region of the antigen binding protein may include an amino acid sequence as shown in SEQ ID NO: 105. For example, the antigen binding protein may include a single-domain antibody MHPD1-A-33 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-33.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 32; the HCDR2 may include an amino acid sequence as shown in SEQ ID NO: 53; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-35 or an antigen binding protein having the same HCDRs 1-3 and H-FRs 1-4 therewith. For example, the heavy chain variable region of the antigen binding protein may include an amino acid sequence as shown in SEQ ID NO: 106. For example, the antigen binding protein may include a single-domain antibody MHPD1-A-35 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-35.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 33; the HCDR2 may include an amino acid sequence as shown in SEQ ID NO: 52; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-36 or an antigen binding protein having the same HCDRs 1-3 and H-FRs 1-4 therewith. For example, the heavy chain variable region of the antigen binding protein may include an amino acid sequence as shown in SEQ ID NO: 107. For example, the antigen binding protein may include a single-domain antibody MHPD1-A-36 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-36.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 34; the HCDR2 may include an amino acid sequence as shown in SEQ ID NO: 46; the HCDR3 may include an amino acid sequence as shown in SEQ ID NO: 64; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-37 or an antigen binding protein having the same HCDRs 1-3 and H-FRs 1-4 therewith. For example, the heavy chain variable region of the antigen binding protein may include an amino acid sequence as shown in SEQ ID NO: 108. For example, the antigen binding protein may include a single-domain antibody MHPD1-A-37 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-37.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 35; the HCDR2 may include an amino acid sequence as shown in SEQ ID NO: 46; the HCDR3 may include an amino acid sequence as shown in SEQ ID NO: 57; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-38 or an antigen binding protein having the same HCDRs 1-3 and H-FRs 1-4 therewith. For example, the heavy chain variable region of the antigen binding protein may include an amino acid sequence as shown in SEQ ID NO: 109. For example, the antigen binding protein may include a single-domain antibody MHPD1-A-38 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-38.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 54; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-39 or an antigen binding protein having the same HCDRs 1-3 and H-FRs 1-4 therewith. For example, the heavy chain variable region of the antigen binding protein may include an amino acid sequence as shown in SEQ ID NO: 110. For example, the antigen binding protein may include a single-domain antibody MHPD1-A-39 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-39.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 54; the HCDR3 may include an amino acid sequence as shown in SEQ ID NO: 3; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-39 H or an antigen binding protein having the same HCDRs 1-3 and H-FRs 1-4 therewith. For example, the heavy chain variable region of the antigen binding protein may include an amino acid sequence as shown in SEQ ID NO: 217. For example, the antigen binding protein may include a single-domain antibody MHPD1-A-39 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-39 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 46; the HCDR3 may include an amino acid sequence as shown in SEQ ID NO: 65; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-40 or an antigen binding protein having the same HCDRs 1-3 and H-FRs 1-4 therewith. For example, the heavy chain variable region of the antigen binding protein may include an amino acid sequence as shown in SEQ ID NO: 111. For example, the antigen binding protein may include a single-domain antibody MHPD1-A-40 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-40.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 36; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-41 or an antigen binding protein having the same HCDRs 1-3 and H-FRs 1-4 therewith. For example, the heavy chain variable region of the antigen binding protein may include an amino acid sequence as shown in SEQ ID NO: 112. For example, the antigen binding protein may include a single-domain antibody MHPD1-A-41 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-41.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 37; the HCDR2 may include an amino acid sequence as shown in SEQ ID NO: 52; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-42 or an antigen binding protein having the same HCDRs 1-3 and H-FRs 1-4 therewith. For example, the heavy chain variable region of the antigen binding protein may include an amino acid sequence as shown in SEQ ID NO: 113. For example, the antigen binding protein may include a single-domain antibody MHPD1-A-42 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-42.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 37; the HCDR2 may include an amino acid sequence as shown in SEQ ID NO: 52; the HCDR3 may include an amino acid sequence as shown in SEQ ID NO: 3; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-42 H or an antigen binding protein having the same HCDRs 1-3 and H-FRs 1-4 therewith. For example, the heavy chain variable region of the antigen binding protein may include an amino acid sequence as shown in SEQ ID NO: 223. For example, the antigen binding protein may include a single-domain antibody MHPD1-A-42 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-42 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 46; the HCDR3 may include an amino acid sequence as shown in SEQ ID NO: 59; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-43 or an antigen binding protein having the same HCDRs 1-3 and H-FRs 1-4 therewith. For example, the heavy chain variable region of the antigen binding protein may include an amino acid sequence as shown in SEQ ID NO: 114. For example, the antigen binding protein may include a single-domain antibody MHPD1-A-43 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-43.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 38; the HCDR2 may include an amino acid sequence as shown in SEQ ID NO: 54; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-44 or an antigen binding protein having the same HCDRs 1-3 and H-FRs 1-4 therewith. For example, the heavy chain variable region of the antigen binding protein may include an amino acid sequence as shown in SEQ ID NO: 115. For example, the antigen binding protein may include a single-domain antibody MHPD1-A-44 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-44.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 38; the HCDR2 may include an amino acid sequence as shown in SEQ ID NO: 54; the HCDR3 may include an amino acid sequence as shown in SEQ ID NO: 3; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-44 H or an antigen binding protein having the same HCDRs 1-3 and H-FRs 1-4 therewith. For example, the heavy chain variable region of the antigen binding protein may include an amino acid sequence as shown in SEQ ID NO: 222. For example, the antigen binding protein may include a single-domain antibody MHPD1-A-44 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-44 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 39; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-45 or an antigen binding protein having the same HCDRs 1-3 and H-FRs 1-4 therewith. For example, the heavy chain variable region of the antigen binding protein may include an amino acid sequence as shown in SEQ ID NO: 116. For example, the antigen binding protein may include a single-domain antibody MHPD1-A-45 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-45.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 39; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 148; the H-FR2 may include an amino acid sequence as shown in SEQ ID NO: 150; the H-FR3 may include an amino acid sequence as shown in SEQ ID NO: 161; the H-FR4 may include an amino acid sequence as shown in SEQ ID NO: 162; for example, the antigen binding protein may include a single-domain antibody MHPD1-A-45 H or an antigen binding protein having the same HCDRs 1-3 and H-FRs 1-4 therewith. For example, the heavy chain variable region of the antigen binding protein may include an amino acid sequence as shown in SEQ ID NO: 223. For example, the antigen binding protein may include a single-domain antibody MHPD1-A-45 H or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-45 H.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 40; the HCDR2 may include an amino acid sequence as shown in SEQ ID NO: 46; the HCDR3 may include an amino acid sequence as shown in SEQ ID NO: 55; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-46 or an antigen binding protein having the same HCDRs 1-3 and H-FRs 1-4 therewith. For example, the heavy chain variable region of the antigen binding protein may include an amino acid sequence as shown in SEQ ID NO: 117. For example, the antigen binding protein may include a single-domain antibody MHPD1-A-46 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-46.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 41; the HCDR2 may include an amino acid sequence as shown in SEQ ID NO: 46; the HCDR3 may include an amino acid sequence as shown in SEQ ID NO: 3; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-47 or an antigen binding protein having the same HCDRs 1-3 and H-FRs 1-4 therewith. For example, the heavy chain variable region of the antigen binding protein may include an amino acid sequence as shown in SEQ ID NO: 118. For example, the antigen binding protein may include a single-domain antibody MHPD1-A-47 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-47.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 46; the HCDR3 may include an amino acid sequence as shown in SEQ ID NO: 66; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-48 or an antigen binding protein having the same HCDRs 1-3 and H-FRs 1-4 therewith. For example, the heavy chain variable region of the antigen binding protein may include an amino acid sequence as shown in SEQ ID NO: 119. For example, the antigen binding protein may include a single-domain antibody MHPD1-A-48 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-48.

In the present application, the antigen binding protein may include a heavy chain variable region, and the heavy chain variable region may include HCDRs 1-3 and H-FRs 1-4. For example, the HCDR1 may include an amino acid sequence as shown in SEQ ID NO: 9; the HCDR2 may include an amino acid sequence as shown in SEQ ID NO: 46; the HCDR3 may include an amino acid sequence as shown in SEQ ID NO: 67; the H-FR1 may include an amino acid sequence as shown in SEQ ID NO: 68; the H-FR2 may include an amino acid sequence as shown in SEQ ID NO: 69; the H-FR3 may include an amino acid sequence as shown in SEQ ID NO: 70; the H-FR4 may include an amino acid sequence as shown in SEQ ID NO: 71; for example, the antigen binding protein may include a single-domain antibody MHPD1-A-49 or an antigen binding protein having the same HCDRs 1-3 and H-FRs 1-4 therewith. For example, the heavy chain variable region of the antigen binding protein may include an amino acid sequence as shown in SEQ ID NO: 120. For example, the antigen binding protein may include a single-domain antibody MHPD1-A-49 or an antigen binding protein (e.g., a single-domain antibody) having the same HCDR3 therewith (e.g., having the same HCDRs 1-3 therewith). In some cases, the antigen binding protein may include an antigen binding protein (e.g., a single-domain antibody) having the same VH as the MHPD1-A-49.

In the present application, the isolated antigen binding protein further includes an Fc region of an immunoglobulin. For example, the present application provides a polypeptide, which includes the antigen binding protein of the present application and the Fc region of the immunoglobulin.

In the present application, the VH of the antigen binding protein is directly or indirectly linked to the Fc region. In the present application, a C-terminus of the VH of the antigen binding protein is directly or indirectly linked to an N-terminus of the Fc region. In the present application, the VH of the antigen binding protein is fused with the Fc region in frame.

In the present application, the VH of the antigen binding protein is linked to the Fc region through a linker. For example, the linker includes a peptide linker. For example, the linker includes a flexible linker.

In the present application, the isolated antigen binding protein includes, sequentially from N-terminus to C-terminus, the VH of the antigen binding protein, the linker and the Fc region.

In the present application, the Fc region includes an Fc derived from IgG1 or an Fc derived from IgG4. For example, the Fc region includes an amino acid sequence as shown in any one of SEQ ID NOs: 121-125.

### Polypeptide and immunoconjugate

In another aspect, the present application provides one or more polypeptides, which may include the isolated antigen binding protein of the present application. For example, the polypeptide may include fusion protein.

In another aspect, the present application provides one or more immunoconjugates, which may include the isolated antigen binding protein of the present application. In some embodiments, the immunoconjugate may further include a pharmaceutically acceptable therapeutic agent.

### Nucleic acid, vector, cell and pharmaceutical composition

In another aspect, the present application further provides one or more isolated nucleic acid molecules, which may encode the isolated antigen binding protein of the present application. For example, each of the one or more nucleic acid molecules may encode the whole or a part (e.g., one or more of HCDRs 1-3 and the heavy chain variable regions) of the antigen binding protein.

The nucleic acid molecules of the present application may be isolated. For example, they may be produced or synthesized by the following methods: (i) amplification *in vitro,* e.g., being produced by amplification through polymerase chain reaction (PCR), (ii) being produced by cloning and recombination, (iii) being purified, for example fractionation by enzymatic digestion and gel electrophoresis, or (iv) being synthesized, for example through chemical synthesis. For example, the isolated nucleic acids may be nucleic acid molecules prepared by a recombinant DNA technology.

In the present application, the nucleic acid encoding the isolated antigen binding protein can be prepared by a variety of methods known in the art, including, but not limited to, utilizing reverse transcription-PCR and PCR to obtain the nucleic acid molecule of the isolated antigen binding protein of the present application.

In another aspect, the present application provides one or more vectors, which include the one or more nucleic acid molecules of the present application. Each vector may include one or more of the nucleic acid molecules. Moreover, the vector may further include other gene(s), e.g., a marker gene that allows the selection of the vector in an appropriate host cell and under appropriate conditions. Moreover, the vector may further include an expression control element that allows the coding region to be properly expressed in an appropriate host. Such a control element is well known by persons skilled in the art. For example, it may include a promoter, a ribosome binding site, an enhancer and other control elements regulating gene transcription or mRNA translation, and the like. In some embodiments, the expression control sequences are regulatable elements. The specific structure of the expression control sequences may vary depending on the function of the species or cell type, but generally includes 5' non-transcribed sequences and 5' and 3' non-translated sequences that are involved in the initiation of transcription and translation respectively, such as TATA box, capped sequences, CAAT sequences, etc. For example, the 5' non-transcribed expression control sequence may include a promoter region which may include a promoter sequence for the transcriptional control of functionally linked nucleic acids. The expression control sequence may further include an enhancer sequence or an upstream activator sequence. In the present application, suitable promoters may include, e.g., promoters for SP6, T3 and T7 polymerases, human U6RNA promoters, CMV promoters and artificially hybrid promoters (e.g., CMV), wherein a part of the promoter may be fused to a part of the gene promoter of other cell proteins (e.g., human GAPDH, glyceraldehyde-3-phosphate dehydrogenase) which may or may not contain an additional intron. The one or more nucleic acid molecules of the present application may be operatively linked to the expression control element.

The vectors may include, e.g., plasmids, cosmids, viruses, phages, or other vectors commonly used in, e.g., genetic engineering. For example, the vectors are expression vectors. For example, the vectors may be viral vectors. The viral vectors may be given to the patient directly (*in vivo*) or indirectly, e.g., by treating the cells with viruses *in vitro* and then administering the treated cells to the patient (*ex vivo*)*.* The viral vector technology is publicly known in the art, and has been described in, e.g., Sambrook et, al. (2001, Molecular Cloning: A Laboratory Manual, Cold Spring Harbor Laboratory, New York) and other manuals of virology and molecular biology. Conventional virus-based systems may include retroviral vectors, lentiviral vectors, adenovirus vectors, adeno-associated virus vectors, and herpes simplex virus vectors for gene transfer. In some cases, the genes can be transferred and integrated into the host genome using methods involving retroviruses, lentiviruses, and adeno-associated viruses, allowing for long-term expression of the inserted genes. Lentiviral vectors are retroviral vectors that can transduce or infect non-dividing cells and typically produce high viral titers. The lentiviral vectors may include long terminal repeats 5'LTR and truncated 3'LTR, RRE, rev response elements (cPPT), central termination sequences (CTS) and/or post-translational regulatory elements (WPRE). The vectors of the present application may be introduced into cells.

In another aspect, the present application provides a cell. The cell may include the isolated antigen binding protein of the present application, the polypeptide, the immunoconjugate, one or more nucleic acid molecules and/or the one or more vectors of the present application. For example, each kind of or each of the cell may include one or one kind of the nucleic acid molecule or vectors of the present application. For example, each kind of or each of the cell may include multiple (e.g., two or more) or multiple kinds of (e.g., two or more kinds of) the nucleic acid molecules or vectors of the present application. For example, the vectors of the present application can be introduced into the host cell, e.g., prokaryotic cell (e.g., bacterial cell), CHO cell, NS/0 cell, HEK293T cell, 293F cell or HEK293A cell, or other eukaryotic cell, such as cell derived from plants, fungi or yeast cell, etc. The vectors of the present application can be introduced into the host cell by methods known in the art, such as electroporation, lipofectine transfection, lipofectamin transfection, etc. For example, the cell may include yeast cell. For example, the cell may include *Escherichia coli* cell. For example, the cell may include mammal cell. For example, the cell may include an immune cell.

The cell may include an immune cell. In some cases, the cell may include immune cell. For example, the cell may include T cell, B cell, natural killer (NK) cell, macrophage, NKT cell, monocyte, dendritic cell, granulocyte, lymphocyte, leukocyte, and/or peripheral blood mononuclear cell.

In another aspect, the present application provides a pharmaceutical composition. The pharmaceutical composition may include the isolated antigen binding protein of the present application, the polypeptide, the immunoconjugate, the isolated nucleic acid molecule, the vector, the cell, and/or a pharmaceutically acceptable adjuvant and/or excipient. In the present application, the pharmaceutically acceptable adjuvant may include a buffer, an antioxidant, a preservative, a low molecular weight polypeptide, a protein, a hydrophilic polymer, an amino acid, a carbohydrate, a chelating agent, a counterion, a metal complex, and/or a nonionic surfactant. Unless incompatible with the cells of the present application, any conventional media or reagents can be considered for use in the pharmaceutical composition of the present application. In the present application, the pharmaceutically acceptable excipient may include additives other than the main ingredients in the pharmaceutical preparation, which can also be called supplements. For example, the excipient may include binder, filler, disintegrating agent, and lubricant in the tablets. For example, the excipient may include wine, vinegar, drug juice, etc. in Chinese medicine pills. For example, the excipient may include the matrix part in semisolid preparations such as ointment and cream. For example, the excipient may include preservative, antioxidant, flavoring agent, fragrance, cosolvent, emulsifier, solubilizer, osmotic pressure regulator, and coloring agent in liquid preparations.

### Detection method, kit, use and method

In another aspect, the present application provides a method for detecting the presence and/or content of PD-1, which may include administering the isolated antigen binding protein or the polypeptide.

In the present application, the method may include an *in vitro* method, an *ex vivo* method, a non-diagnostic or non-therapeutic method.

For example, the method may include a non-diagnostic method for detecting the presence and/or content of PD-1, which may include the following steps:
1) contacting a sample with the antigen binding protein of the present application; and
2) determining the presence and/or expression level of PD-1 in the sample obtained from the subject by detecting the presence and/or content of the antigen binding protein bound to the sample.

In another aspect, the present application provides a kit, which may include the isolated antigen binding protein or the polypeptide.

In the present application, the kit may further include an instruction for describing the method for detecting the presence and/or content of PD-1. For example, the method may include an *in vitro* method, an *ex vivo* method, a non-diagnostic or non-therapeutic method.

In another aspect, the present application provides use of the isolated antigen binding protein or the polypeptide in the preparation of a kit used in a method for detecting the presence and/or content of PD-1. For example, the method may include an *in vitro* method, an *ex vivo* method, a non-diagnostic or non-therapeutic method.

In another aspect, the present application provides use of the isolated antigen binding protein and/or the polypeptide in the preparation of a drug for preventing and/or treating a disease or a disorder.

In another aspect, the present application provides the isolated antigen binding protein, the polypeptide, the immunoconjugate, the isolated nucleic acid molecule, the vector, the cell and/or the pharmaceutical composition, which are used for preventing, relieving and/or treating a disease or a disorder.

For example, the disease or disorder may include a tumor. For example, the tumor may include a solid tumor. For example, the tumor may include a blood tumor. For example, the tumor may include a tumor associated with the expression of PD-L1. For example, the heavy chain may include a tumor associated with the upregulation of the PD-L1 expression. For example, the tumor may be selected from the group consisting of melanoma, lung cancer, head and neck squamous cell carcinoma, lymphoma, hepatocellular carcinoma, renal cell carcinoma, urothelial carcinoma, colorectal cancer, and breast cancer.

In another aspect, the present application provides a method for preventing and/or treating a disease or a disorder, which may include administering to a subject in need an effective amount of the isolated antigen binding protein, the polypeptide, the immunoconjugate, the isolated nucleic acid molecule, the vector, and/or the cell.

For example, the disease or disorder may include a tumor. For example, the tumor may include a solid tumor. For example, the tumor may include a blood tumor. For example, the tumor may include a tumor associated with the expression of PD-L1. For example, the heavy chain may include a tumor associated with the upregulation of the PD-L1 expression. For example, the tumor may be selected from the group consisting of melanoma, lung cancer, head and neck squamous cell carcinoma, lymphoma, hepatocellular carcinoma, renal cell carcinoma, urothelial carcinoma, colorectal cancer, and breast cancer.

In another aspect, the present application provides a method for inhibiting the interaction between PD-1 and PD-L1, which includes administering to a subject in need an effective amount of the isolated antigen binding protein, the polypeptide, the immunoconjugate, the isolated nucleic acid molecule, the vector, and/or the cell. The method may be an *ex vivo* or *in vitro* method. In the present application, the method may include contacting a biological sample with the antigen binding protein of the present application and/or PD-L1 under a condition allowing the binding of the antigen binding protein and/or PD-L1 to PD-1, detecting whether a complex is formed between the antigen binding protein and PD-1, and detecting whether a complex is formed between PD-1 and PD-L1.

In another aspect, the present application provides the isolated antigen binding protein, the polypeptide, the immunoconjugate, the isolated nucleic acid molecule, the vector, the cell and/or the pharmaceutical composition, which are used to inhibit the interaction between PD-1 and PD-L1.

In another aspect, the present application provides use of the isolated antigen binding protein and/or the polypeptide in the preparation of a drug for inhibiting the interaction between PD-1 and PD-L1.

Without intending to be limited by any theory, the embodiments below are intended only to illustrate the antigen binding protein, the preparation method and the use of the present application and are not intended to limit the inventive scope of the present application.

### Example

### Example 1. Immunization of Alpaca

A healthy, female, adult alpaca was immunized with the recombinant human PD-1 protein. The priming involved multiple-site subcutaneous injection of 1 mg of the recombinant human PD-1 protein emulsified with an equivalent volume of Freund's Complete Adjuvant; and the boosting involved multiple-site subcutaneous injection of 0.5 mg of the recombinant human PD-1 protein emulsified with an equivalent volume of Freund's Incomplete Adjuvant. A total of 3 booster doses were performed.

### Example 2. Construction of Alpaca Immunization Library

2.1 At the end of immunization, the peripheral blood of the alpaca was collected. Lymphocytes were isolated using a Lymphocyte Isolation Liquor (Solarbio, Cat.P8900); and total RNAwas extracted using a TRIzol^{™} Reagent (Thermo Fisher Scientific, Cat. 15596018).

2.2 cDNA was obtained by reverse transcription using a PrimeScript^{™} II 1st Strand cDNA Synthesis Kit (TAKARA, Code No.6210B), and VHH gene was amplified by nested PCR.

2.3 VHH gene fragments were recovered using a Gel Purification Kit (TAKARA, Code No.9761), digested with restriction endonuclease sfil (TAKARA, Code No.1244A), and then cloned into the phasmid vector pComb 3XSS. The constructed cloned product was transformed into *E.coli* TG1 electroporation-competent cells to give a PD-1 single domain antibody library.

2.4 The capacity of the library was measured as 3.2 × 10⁸ using a plate gradient dilution method, and the PCR results of colony show that the clone rate of the library was 98%. An appropriate amount of library bacteria liquid (with the bacteria number of about 3.2 × 10⁹) was inoculated into 100 mL of LB medium (containing ampicillin) and cultured to middle logarithmic phase. An appropriate amount of helper phage M13KO7 was added for infection. After 60 minutes, the medium was replaced with 200 mL of 2 × YT (containing ampicillin and kanamycin), and cultured under violent shaking at 30°C for 12 hrs. A supernatant was collected by centrifuge, and an appropriate amount of 20% PEG-NaCl solution was added to precipitate the phage. The precipitate was re-suspended in an appropriate amount of PBS to give the phage display library which can be directly used in subsequent screening.

### Example 3. Screening of Nanobody

PD-1 nanobody was screened using solid phase affinity screening method with total of three rounds of screening.

3.1 Coating: Microplate was coated with recombinant human PD-1 protein at concentration of 100 µg/mL in Round 1, 50 µg/mL in Round 2, and 20 µg/mL in Round 3 at 4°C overnight.

3.2 Blocking: The plate was blocked with 3% ovalbumin in Rounds 1 and 3 and 3% bovine serum albumin in Round 2 at 37°C for 1 hr.

3.3 Binding and washing: In Round 1 of screening, after the removal of blocking solution, 100 µL of phage display library of nanobody constructed in Embodiment 2 was added into the microplate, incubated to bind at 37°C for 1 hr., and then the unbounded library was removed. The microplate was washed with PBST and PBS (5 times each) to remove unbounded phages or phages with low binding; in Round 2, the microplate was washed with PBST and PBS 20 times each; and in Round 3, the microplate was washed with PBST and PBS 10 times each.

3.4 Elution of specific phage: 100 µL of glycine hydrochloride buffer (pH 2.2) was added to the microplate to dissociate the specific phage. After slightly shaking for 10 min, the eluent was collected.

3.5 Titer measurement and amplification of eluate: *E. coli* TG1 in the logarithmic phase was infected with the eluate. A small amount of infected *E.coli* TG1 was subject to gradient dilution, and an appropriate amount of gradient diluted solution was uniformly coated onto the LB solid medium (plate dilution method). After 12-14 hrs., the plate was counted to calculate the amount of phage eluted. The remainder was subject to amplification to give a specific phage-enriched library for the next round of screening.

3.6 Identification: 93 clones were randomly picked from each of the plates used to measure the eluate titer in the aforesaid Rounds 2 and 3, and amplified to obtain phages, which were identified by ELISA. The method includes coating with recombinant human PD-1 protein (with a coating concentration of 2 µg/mL), blocking with 5% skim milk, adding the phage to be identified, binding at 37°C for 1 hr., washing and adding Goat anti-Llama IgG (H+L) Secondary Antibody [HRP], binding at 37°C for 1 hr., washing and adding the TMB substrate for development, and reading OD₄₅₀ by a microplate reader. Positive control wells (the enriched library in each round), negative control (helper phage M13KO7) and blank well (PBS) were set. It will be judged as positive if the OD value of the well to be measured is more than 3 times of the negative control. The clones corresponding to the positive wells were preserved, extracted for plasmid, sequenced, and aligned after translating the DNA sequence into an amino acid sequence. The clones with the same HCDR1, HCDR2 and HCDR3 sequences were regarded as the same antibody strain, while the clones with different HCDR sequences were regarded as different antibody strains.

### Example 4. Expression and Purification of VHH

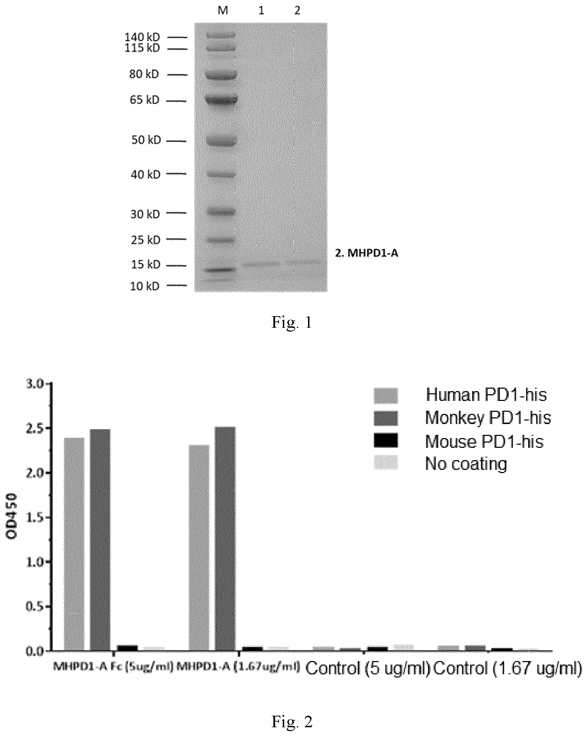

The positive clones screened in Embodiment 3 were transformed into Top10F', and induced by IPTG for VHH expression. Cell precipitation was collected by centrifugation, and ultrasonicated to collect the supernatant. The supernatant was purified by TALON^{®} Metal Affinity Resins and the buffer was replaced by a phosphate buffer. The purified VHH was quantified by measuring OD280 value and stored at 4°C for later use. Of those, the SDS PAGE of the screened VHH which has higher affinity to antigen (human recombinant PD-1) and can compete with the human recombinant PD-L1 to bind to the human recombinant PD-1 is shown in Fig. 1. The resultant VHH is named MHPD1-A.

### Example 5. Competitive Binding ELISA of VHH with Recombinant Human PD-L1 to Antigen Protein

Ninety-six-well plates were coated with recombinant human PD-1 antigen (2 µg/mL) at 4°C overnight. The plates were blocked with 1% casein. The MHPD1-A sample was subject to 2-folded gradient dilution with 0.1% BSA/PBS with an initial concentration of 20 µg/mL. Then 4 µg/mL of recombinant human PD-L1-Fc and different concentrations of VHH were added into the 96-well plates coated with the antigen (recombinant human PD-1 protein), and incubated at room temperature for 1 hr. The plates were washed with PBST between incubations. Horseradish peroxidase-labelled anti-human IgG secondary antibody (1:5000, containing 1% casein) was used for detection, and Graphpad Prism software was used to fit the curve and calculate the EC₅₀ value, with the results shown in Table 1. The results show that MHPD1-A can effectively inhibit the binding of human recombinant PD-L1 to human recombinant PD-1.

**Table 1. Results of Competitive Inhibition of VHH to Antigen Protein**

| Antibody Name | EC₅₀ (µg/mL) |
|---|---|
| MHPD1-A | 1.71 |

### Example 6. Expression and Purification of Fusion Protein

pTT5 recombinant vector comprising nucleic acid sequences encoding MHPD1-A and human Fc fragment (the C-terminal of VHH directly connected to the N-terminal of human Fc hinge region) was synthesized, and the liposome-DNA complex was prepared by steps of diluting 15 µg of plasmid DNA with Opti-MEM^{®} I to a total volume of 0.5 mL; diluting 30 µL of 293fectin^{™} reagent with Opti-MEM^{®} I to a total volume of 0.5 mL; mixing them gently; and incubating them at room temperature for 5 min. The diluted pTT5 recombinant vector was added into the diluted 293fectin^{™}, gently mixed to uniform, and incubated at room temperature for 30 min. The mixture was added into a shake flask containing 1 × 10⁶ cells/mL, and incubated at 37°C in a cell incubator with 8% CO₂ on an orbital oscillator rotating at 125 rpm. On Day 4 after transfection, the supernatant was harvested, purified by Protein A agarose gel (Pierce^{™}), and the buffer was replaced by a phosphate buffer. The resultant fusion protein was named MHPD1-AFc.

### Example 7. Species Cross-validation of Fusion Protein with Human, Monkey and Mouse PD-1

Ninety-six-well Greiner plates were coated with 2 µg/mL of recombinant human PD-1-his protein, recombinant monkey PD-1-his protein and recombinant mouse PD-1-his protein at 4°C overnight. The next day, the plates were blocked with 1% casein. Then, 5 µg/mL and 1.67 µg/mL nanobody-Fc fusion protein samples were added into the antigen-coated plates and incubated for 2 hrs. Then, the plates were washed with PBST. Anti-human IgG-HRP (diluted in 1% casein at 1:5000) was added and incubated for 1 hr., and binding detection was performed. As shown in Fig. 2, MHPD1-A Fc can bind to the recombinant human PD-1-his and the recombinant monkey PD-1-his, but has no binding activity to the recombinant mouse PD-1-his.

### Example 8. Detection of Affinity of Fusion Protein to Recombinant Human PD-1

About 250RU of recombinant human PD-1-his protein was coupled to a CM5 chip at 25°C using an amine coupling kit (GE Healthcare, Cat No.BR100050) according to standard amine coupling procedure. Two-fold serially diluted MHPD1-A Fc samples were injected at a flow rate of 30 µL/min at 25°C (five concentrations were detected). The binding time was 120 s, and the dissociation time was 600 s. The curve was fitted with 1:1 Langmuir binding model using Biacore^{®}8K analysis software (BIAevaluation) to determine the kinetic constants thereof, such as, association rate constant, dissociation rate constant, equilibrium dissociation constant, etc.

The results show that MHPD 1-A Fc has higher affinity with the recombinant human PD-1, and the kinetic constants of MHPD1-A Fc binding to the recombinant human PD1-his are shown in Table 2.

**Table 2. Kinetic Constants of Nanobody-Fc Fusion Protein Binding to hPD-1-his**

| **Analyte** | **ka (1/Ms)** | **kd (1/s)** | **Rmax (RU)** | **KD** | **Chi² (RU²)** |
|---|---|---|---|---|---|
| MHPD1-AFc | 4.60E+05 | 1.95E-05 | 85.6 | 4.25E-11 | 1.57E-01 |

### Example 9. Competitive Binding ELISA of Fusion Protein with Recombinant Human PD-L1 to Recombinant Human PD-1

Ninety-six-well plates were coated with recombinant human PD-L1 (C-Fc, 0.2 µg/well) at 4°C overnight. The plates were blocked with 1% casein. The MHPD1-A was subject to 2-fold gradient dilution with 0.1% BSA/PBS with an initial concentration of 10 µg/mL. Then 0.5 µg/mL of recombinant human PD-1 (C-Fc & C-Avi & C-6 × His) and different concentrations of MHPD1-A Fc were added into the 96-well plates coated with the antigen, and incubated at room temperature for 1 hr. The plates were washed with PBST between incubations. Horseradish peroxidase-labelled streptavidin (1:10000) was used for detection, and Graphpad Prism software was used to fit the curve and calculate the EC₅₀ value, with the results shown in Table 3.

The results show that the tested MHPD1-A Fc can competitively inhibit the binding of the recombinant human PD-L1 to the recombinant human PD-1, and the activity is comparable with or superior to that of the positive control Nivolumab.

**Table 3. Competitive Binding Activity of Fusion Protein**

| Antibody Name | EC₅₀ (µg/mL) |
|---|---|
| MHPD1-AFc | 0.499 |
| Nivolumab | 0.593 |

### Example 10. Binding Activity of Fusion Protein to HEK293 Cells Overexpressing Human PD-1

HEK293 cells overexpressing the human PD-1 were cultured in a DMEM medium containing 10% FBS in a CO₂ incubator (37°C, 5% CO₂). The cells were harvested and washed once with test buffer. Cells were counted and detected for viability. Then 3 × 10⁵ viable cells were added into individual tubes, and 100 µL of different concentrations of MHPD1-AFc (3000 ng/mL, 1000 ng/mL, 300 ng/mL, 100 ng/mL, 30 ng/mL, 10 ng/mL, 3 ng/mL, 1 ng/mL and 0.3 ng/mL) were added and incubated at 4°C for 1hr. The cells were washed with washing buffer, and 100 µL of PE-anti-human IgG was added in accordance with the instruction and incubated in dark at 4°C for 1 hr. The cells were washed three times with washing buffer, and individual samples were re-suspended in 200 µL of PBS and detected by flow cytometer. The data were analyzed using FCS Express 6 Plus and GraphPad Prism 5 software.

The results show that MHPD1-A Fc can bind to the HEK293 cells overexpressing human PD-1. The EC₅₀ of MHPD1-A Fc binding to the HEK293 cells overexpressing human PD-1 is shown in Table 4.

**Table 4. Binding Activity of Fusion Protein to HEK293 Cells Overexpressing Human PD-1**

| | |
|---|---|
| Fusion protein | EC₅₀ (µg/mL) |
| MHPD1-AFc | 0.2448 |

### Example 11. Competitive Binding of Fusion Protein with Human PD-L1 to HEK293 Cells Overexpressing Human PD-1

In a CO₂ incubator (37°C, 5% CO₂), HEK293 cells overexpressing human PD-1 were cultured in a DMEM medium containing 10% FBS and 1 mg/mL of G418. The cells were harvested and washed once with test buffer. The cells were counted and detected for viability. 3 × 10⁵ viable cells (with cell viability of ≥ 95%) were added into individual tubes. 1.0 µg/mL of biotinylated human PD-L1 protein and the serially diluted MHPD1-A Fc (with concentrations from 0.03 µg/mL to 10 µg/mL) were mixed at room temperature and incubated for 30 min, respectively. The mixtures were added into centrifuge tubes containing 3 × 10⁵ viable cells, and incubated at 4°C for 1 hr. The cells were washed with washing buffer, and 100 µL of PE-SA was added in accordance with the instruction and incubated at 4°C for 1 hr. The cells were washed three times with washing buffer. Individual samples were re-suspended in 100 µL of PBS and detected by flow cytometer. The data were analyzed using FCS Express 6 Plus and GraphPad Prism 5 software.

The results show that MHPD1-A Fc can competitively inhibit the binding of the recombinant human PD-L1 to HEK293 cells overexpressing hPD-1, and the competitive binding activity of MHPD1-A Fc is slightly superior to that of the positive control Nivolumab. The EC₅₀ values of MHPD1-A Fc competing with human PD-L1 to bind to HEK293/hPD1 are shown in Table 5.

**Table 5. Fusion Protein Competing with Human PD-L1 to Bind HEK293 Cells Overexpressing Human PD-1**

| Fusion protein | EC₅₀ (µg/mL) |
|---|---|
| MHPD1-AFc | 0.549 |
| Nivolumab | 0.580 |

### Example 12. Detection of Affinity Kinetics of Fusion Protein and Pembrolizumab to Antigen Protein

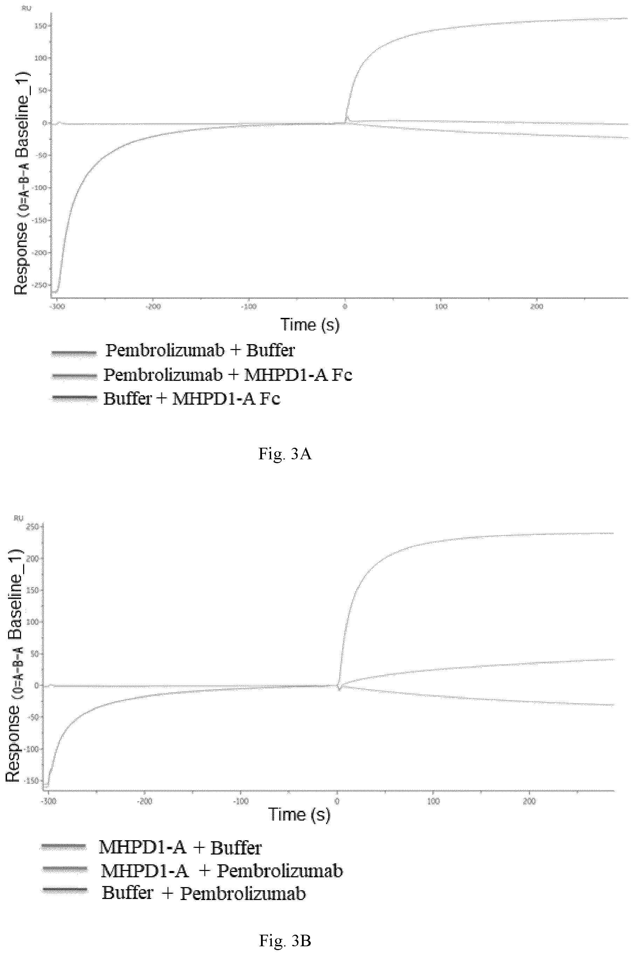

Affinity kinetic detection was performed using Biacore^{®} 8K (GE Healthcare): Recombinant human PD-1-his protein was coupled to a CM5 chip (GE Healthcare, Cat No. BR100530) at 25°C using an amine coupling kit (GE Healthcare, Cat No.BR100050) according to standard amine coupling procedures. In one trial, Pembrolizumab (100 nM) and MHPD1-A Fc (2 µM), or Pembrolizumab (100 nM) and a buffer, or a buffer and MHPD1-A Fc (2 µM) were sequentially injected at a flow rate of 30 µL/min at 25°C. The binding time for each sample is 300 s, and the affinity kinetic curve is shown in Fig. 3A. In another trial, MHPD1-A Fc (2 µM) and Pembrolizumab (100 nM), or MHPD1-A Fc (2 µM) and a buffer, or a buffer and Pembrolizumab (100 nM) were sequentially injected at a flow rate of 30 µL/min at 25°C. The binding time for each sample is 300 s, and the affinity kinetic curve is shown in Fig. 3B. Based on the above results, it is speculated that the epitopes for MHPD1-A Fc and Pembrolizumab binding in PD1-His protein are the same or overlapped.

### Example 13. Construction of VHH Variants

Through database sequence analysis of MHPD1-A sequence, a series of VHH variant sequences were designed by mutating some amino acid sites of HCDR1-3 based on structural prediction and experience. According to the designed VHH variant sequences, the whole gene synthesis was performed (His tag). The plasmid pcDNA3.4 was digested with NotI/XbaI (NEB, Cat. R0189L, R0145L) and recombined with the VHH variant gene sequences. The recombinant plasmid was transformed into TOP10 competent cell, coated and cultured at 37°C for 16-20 h. Single colonies in the plate were picked for colony PCR and identified by electrophoresis for the positive clones. The positive clones were selected for sequencing, and the clones with correct sequence were expanded to extract plasmids. HEK293 cells were transiently transfected with liposome transfection reagent, placed in a cell incubator containing 5% CO₂ at 37°C, and incubated on an orbital oscillator rotating at 125rpm. On Day 5, the supernatant was collected by centrifugation, purified by Ni affinity chromatography column (Ni Smart Beads 6FF, GE #17531802), and dialyzed to replace the buffer with a phosphate buffer to obtain a series of VHH variants, which were named MHPD1-A-4, MHPD1-A-7, MHPD1-A-8, MHPD1-A-13, MHPD1-A-15, MHPD1-A-24, MHPD1-A-25, MHPD1-A-30, MHPD1-A-32, MHPD1-A-39, MHPD1-A-42, MHPD1-A-44 and MHPD1-A-45, respectively.

### Example 14. Competitive Binding ELISA of VHH Variants with Recombinant Human PD-L1 to Antigen Protein

Ninety-six-well plates were coated with recombinant human PD-1 protein (2 µg/mL) at 4°C overnight. The plates were blocked with 1% casein. The VHH variants were subject to 2-folded gradient dilution with 0.1% BSA/PBS with an initial concentration of 20 µg/mL. Then 4 µg/mL of recombinant human PD-L1-Fc and different concentrations of VHH variants were added into the 96-well plates coated with the antigen (recombinant human PD-1 protein), and incubated at room temperature for 1 hr. The well plates were washed with PBST between incubations. Horseradish peroxidase-labelled anti-human IgG secondary antibody (1:5000, containing 1% casein) was used for detection. After analysis, the results show that the VHH variants can effectively inhibit the recombinant human PD-L1 from binding to the recombinant human PD-1.

### Example 15. Construction of Fusion Protein Containing VHH variant

VHH variants with high affinity were selected for synthesizing full-length sequences comprising their gene sequences and Fc sequences. pcDNA3.4 plasmid was digested with NotI/XbaI (NEB, Cat. R0189L, R0145L), and recombined with the full-length sequences. The recombinant plasmid was transformed into TOP10 competent cell, coated and cultured at 37°C for 16-20 h. Single colonies in the plate were picked for colony PCR and identified by electrophoresis for the positive clones. The positive clones were selected for sequencing, and the clones with the correct sequence were expanded to extract plasmids. HEK293 cells were transiently transfected with liposome transfection reagent, placed in a cell incubator containing 5% CO₂ at 37°C, and incubated on an orbital oscillator rotating at 125rpm. On Day 5, the supernatant was collected by centrifugation, purified by Protein A affinity chromatographic column, and dialyzed to replace the buffer with a phosphate buffer, thus fusion proteins containing the VHH variants were obtained.

### Example 16. Competitive Binding ELISA of Fusion Protein containing VHH Variants with Recombinant Human PD-L1 to the Antigen Protein

Ninety-six-well plates were coated with recombinant human PD-L1 (C-Fc, 0.2 µg/well) at 4°C overnight. The plates were blocked with 1% casein. The VHH variant fusion proteins were subject to 2-fold gradient dilution with 0.1% BSA/PBS with an initial concentration of 10 µg/mL. Then 0.5 µg/mL of recombinant human PD-1 (C-Fc & C-Avi & C-6 × His) and different concentrations of VHH variant fusion proteins were added into the 96-well plates coated with the antigen, and incubated at room temperature for 1 hr. The plates were washed with PBST between incubations. Horseradish peroxidase-labelled streptavidin (1:10000) was used for detection. After analysis, the results show that the VHH variant fusion protein can effectively inhibit the recombinant human PD-L1 from binding to the recombinant human PD-1.

### Example 17. Humanization of Nanobody and Detection of Binding Activity of Humanized Nanobody-Fc Fusion Protein to Antigen Protein

Through database sequence analysis on the sequence of the original alpaca-derived antibody MHPD1-A, the human sequence Germeline to be referenced was determined, and the humanized sequence was designed. After the first round of humanization, the degree of humanization varied from 86.44% to 90.68%. They were constructed into nanobody-Fc fusion proteins, which were transiently expressed in ExpiCHO-s, and then a second round of humanization was performed according to the degree of humanization and affinity knot. After the second round of humanization, the degrees of humanization are all greater than 90%. They were constructed into nanobody-Fc fusion proteins, which were transiently expressed in ExpiCHO-s, and verified for their binding activity.

The binding activity of nanobody-Fc fusion protein with different degrees of humanization after two rounds of humanization to the antigen protein (human PD-1) was verified by ELISA method. 96-well ELISA plates were coated with 2 µg/mL of recombinant human PD-1-his antigen protein at 30 µL/well at 4°C overnight. The plates were washed three times with PBST. 5% PBS-Milk was added and incubated at room temperature for 2 h. The plates were washed three times with PBST. All the nanobody-Fc fusion proteins were subject to 3-fold gradient dilutions with an initial concentration of 10 µg/mL, added into each well at 30 µL/well, and incubated at room temperature for 1 hr. The plates were washed three times with PBST. The secondary antibody Goat-Anti-Human-IgG-Fc-HRP (purchased from Sigma) was diluted at 1:8000, added into each well at 30 µL/well, and incubated at room temperature for 50 min. The plates were washed three times with PBST. TMB (purchased from SurModics) was added into each well at 30 µL/well and developed at room temperature in dark for 1 min to 5 min. 2 M hydrochloric acid was added at 30 µL/well to stop the reaction. The OD450 absorbance value was read by a microplate reader. Graphpad Prism software was used to fit the curve and calculate the EC₅₀ value, as shown in Table 6. The results show that the humanized nanobody-Fc fusion protein can still effectively bind to the antigen protein, and the affinity of some humanized nanobody-Fc fusion protein against the antigen protein is comparable with or superior to that of the parent antibody.

**Table 6. Binding Activity of Humanized Nanobody-Fc Fusion Protein to Antigen Protein Human PD-1**

| ELISA Results of First Round of Humanization | | ELISA Results of Second Round of Humanization | |
|---|---|---|---|
| Antibody Code | EC₅₀ (µg/mL) | Antibody Code | EC₅₀ (nM) |
| MHPD1-A Fc | 0.14 | MHPD1-A Fc | 0.34/0.41 |
| MHPD1-A-H1 Fc | 0.24 | MHPD1-A-H9 Fc | 0.23/0.36 |
| MHPD1-A-H2 Fc | 0.22 | MHPD1-A-H12 Fc | 4.32 |
| MHPD1-A-H3 Fc | 0.16 | MHPD1-A-H14 Fc | 1.88 |
| MHPD1-A-H5 Fc | 0.35 | MHPD1-A-H15 Fc | 2.15 |
| MHPD1-A-H6 Fc | 2.91 | MHPD1-A-H16 Fc | 2.29 |
| MHPD1-A-H7 Fc | 2.54 | MHPD1-A-H17 Fc | 0.64 |
| MHPD1-A-H8 Fc | 0.20 | MHPD1-A-H18 Fc | 1.29 |
| MHPD1-A-H9 Fc | 0.15 | MHPD1-A-H19 Fc | 0.55 |
| MHPD1-A-H10 Fc | 0.32 | | |

### Example 18. Affinity Maturation of Humanized Nanobody and Detection of Binding Activity of Nanobody-Fc Fusion Protein to Antigen (First Round)

The VHH of parent antibody MHPD1-A-H19 was cloned into the phage display vector of the secondary party. A series of primers were designed to construct an affinity-matured phage display library by performing single-point or continuous three-point mutation on HCDR. Immunotube screening (i.e., solid phase screening) was used. The immunotube was coated with antigen protein hPD-1-His, and the affinity-matured phage display library was added for incubation, washing and elution. After 2-3 rounds of screening, specific monoclonal antibodies with high affinity were enriched. After affinity maturation screening, candidate antibodies with high affinity to hPD-1-His antigen were screened at ELISA level. According to the affinity and sequence of the VHH, 14 candidate molecules were selected to construct nanobody-Fc fusion protein.

The binding activity of nanobody-Fc fusion protein to antigen protein (hPD-1-his) after affinity maturation was verified by ELISA method: 96-well ELISA plates were coated with 2 µg/mL of recombinant human PD-1-his antigen protein at 30 µL/well at 4°C overnight. The plates were washed three times with PBST. 5% PBS-Milk was added and incubated at room temperature for 2 h. The plates were washed three times with PBST. All the nanobody-Fc fusion proteins were subject to 3-fold gradient dilutions with an initial concentration of 10 µg/mL, added into each well at 30 µL/well, and incubated at room temperature for 1 hr. The plates were washed three times with PBST. The secondary antibody Goat-Anti-Human-IgG-Fc-HRP was diluted at 1:8000, added into each well at 30 µL/well, and incubated at room temperature for 50 min. The plates were washed nine times with PBST. TMB was added into each well at 30 µL/well and developed at room temperature in dark for 1 min to 5 min. 2 M hydrochloric acid was added at 30 µL/well to stop the reaction. The OD450 absorbance value was read by a microplate reader. Graphpad Prism software was used to fit the curve and calculate the EC₅₀ value (see Table 7). The results show that after affinity maturation, the binding activity of multiple nanobody-Fc fusion proteins to antigen protein human PD-1 was superior to that of the parent MHPD1-A-H19 Fc.

**Table 7. Binding Activity of Affinity-Matured Nanobody-Fc Fusion Protein to Antigen Protein Human PD-1**

| Antibody Code | EC₅₀ (nM) | Antibody Code | EC₅₀ (nM) |
|---|---|---|---|
| MHPD1-A-H19 Fc | 0.369 | MHPD1-A-8 H Fc | 0.169 |
| MHPD1-A-1 H Fc | 0.613 | MHPD1-A-9 H Fc | 0.163 |
| MHPD1-A-2 H Fc | 0.553 | MHPD1-A-10 H Fc | 0.200 |
| MHPD1-A-3 H Fc | 0.251 | MHPD1-A-11 H Fc | 0.222 |
| MHPD1-A-4 H Fc | 0.169 | MHPD1-A-12 H Fc | 0.311 |
| MHPD1-A-5 H Fc | 0.157 | MHPD1-A-13 H Fc | 0.140 |
| MHPD1-A-6 H Fc | 0.160 | MHPD1-A-14 H Fc | 1.218 |
| MHPD1-A-7 H Fc | 0.135 | | |

### Example 19. Competitive Binding ELISA of Affinity-Matured Nanobody-Fc Fusion Protein with Recombinant Human PD-L1 to Recombinant Human PD-1

Ninety-six-well plates were coated with recombinant human PD-L1-mFc (8 µg/mL) at 4°C overnight. The plates were washed three times with PBST. 5% PBS-Milk was added and incubated at room temperature for 1 hr. All the nanobody-Fc fusion proteins were subject to 3-fold gradient dilutions with an initial concentration of 10 µg/mL, added into each well at 30 µL/well, and incubated at room temperature for 1 hr. Then 0.5 µg/mL of recombinant human PD-1-Fc-Biotin was added into the 96-well plates coated with the antigen at 30 µL/well and incubated at room temperature for 1 hr. The well plates were washed with PBST between incubations. Horseradish peroxidase-labelled streptavidin (1:10000) was used for detection, and Graphpad Prism software was used to fit the curve and calculate the IC₅₀ value, as shown in Table 8. The results show that after the affinity maturation, the competitive inhibitory activities of multiple nanobody-Fc fusion proteins against the binding of the antigen protein human PD-1 to the ligand human PD-L1 were increased as compared with that before the affinity maturation, and superior to that of the positive control.

**Table 8. Competitive Inhibitory Activity of Affinity-Matured Nanobody-Fc Fusion Protein Against the Binding of Recombinant Human PD-L1 to Human Recombinant PD-1**

| Antibody Code | IC₅₀ (nM) | Antibody Code | IC₅₀ (nM) |
|---|---|---|---|
| MHPD1-A-H19 Fc | 1.310 | MHPD1-A-8 H Fc | 0.323 |
| MHPD1-A-1 H Fc | 0.572 | MHPD1-A-9 H Fc | 0.256 |
| MHPD1-A-2 H Fc | 0.419 | MHPD1-A-10 H Fc | 0.287 |
| MHPD1-A-3 H Fc | 0.275 | MHPD1-A-11 H Fc | 0.365 |
| MHPD1-A-4 H Fc | 0.281 | MHPD1-A-12 H Fc | 0.293 |
| MHPD1-A-5 H Fc | 0.306 | MHPD1-A-13 H Fc | 0.395 |
| MHPD1-A-6 H Fc | 0.407 | MHPD1-A-14 H Fc | 0.852 |
| MHPD1-A-7 H Fc | 0.316 | Pembrolizumab | 0.410 |
| Nivolumab | 0.402 | | |

### Example 20. Detection of Biological Activity of Nanobody-Fc Fusion Protein

Using Jurkat-NFAT-PD1 transgene cells as effector cells, and Hep3B-OS8-PDL1 as target cells, the biological activity of the nanobody-Fc fusion protein blocking the PD1/PDL1 binding was detected by Luciferase Assay System. Hep3B-OS8-PDL1 cells were re-suspended in PRMI1640 medium with 10% FBS, inoculated into a 96-well cell culture plate, and placed in a cell incubator for culturing overnight. The next day, the medium was removed from the cell culture plate. 50 uL of pre-made mediums containing different concentrations of nanobody-Fc fusion proteins and 50 uL of Jurkat-NFAT-PD1 cell suspension were added into individual wells, placed in a carbon dioxide incubator (37°C, 5% CO₂) and incubated for 6 hrs. The cell culture plates were taken out and equilibrated at room temperature for 5-10 min. 100 uL of luciferase reporter gene detection reagent (One-Glo^{™} Luciferase assay system, Promega) was added into individual wells, incubated for at least 3 min, and then detected with microplate reader for fluorescence signal (see Table 9). The results show that the antibodies A-3 H Fc, A-4 H Fc, A-7 H Fc after affinity maturation can effectively block the inhibition of PD-1/PD-L1 binding on downstream reporter gene fluorescence signal, and the biological activity is comparable with or superior to the positive control Pembrolizumab.

**Table 9. Biological Activity of Affinity-Matured Nanobody-Fc Fusion Protein**

| Antibody Code | EC₅₀ (nM) |
|---|---|
| MHPD1-A-3 H Fc | 0.078 |
| MHPD1-A-4 H Fc | 0.090 |
| MHPD1-A-7 HFc | 0.070 |
| Pembrolizumab | 0.091 |

### Example 21. Affinity Maturation of Nanobody and Detection of Competitive Binding Activity of Nanobody-Fc Fusion Protein to Human PD-1 and Human PD-L1 to Human PD-1 (Round 2)

The VHH of parent MHPD1-A-7 H was cloned into the phage display vector of the secondary party. A series of primers were designed to construct an affinity-matured phage display library by performing single-point or continuous three-point mutation on HCDR. Immunotube screening (i.e., solid phase screening) was used. The immunotube was coated with antigen protein hPD-1-His, and the affinity-matured phage display library was added for incubation, washing and elution. After 2-3 rounds of screening, specific monoclonal antibodies with high affinity were enriched. After affinity maturation screening, candidate antibodies with high affinity to hPD-1-His antigen were screened at ELISA level. According to the affinity and sequence of VHH, 29 candidate molecules were selected to construct nanobody-Fc fusion protein.

The binding activity of nanobody-Fc fusion protein to antigen protein (hPD-1-his) after affinity maturation was verified by ELISA method: 96-well ELISA plates were coated with 2 µg/mL of recombinant human PD-1-his antigen protein at 30 µL/ well at 4°C overnight. The plates were washed three times with PBST. 5% PBS-Milk and incubated at room temperature for 2 h. The plates were washed three times with PBST. All the nanobody-Fc fusion proteins were subject to 3-folded gradient dilution with an initial concentration of 10 µg/mL, added into each well at 30 µL/well, and incubated at room temperature for 1 hr. The plates were washed three times with PBST. The secondary antibody Goat-Anti-Human-IgG-Fc-HRP was diluted at 1 :8000, added into each well at 30 µL/well, and incubated at room temperature for 50 min. The plates were washed nine times with PBST. TMB was added into each well at 30 µL/well and developed at room temperature in dark for 1 min to 5 min. 2 M hydrochloric acid was added at 30 µL/well to stop the reaction. The OD450 absorbance value was read by a microplate reader. Graphpad Prism software was used to fit the curve and calculate EC₅₀ values. As shown in Table 10, multiple nanobody-Fc fusion proteins have comparable binding activity to the antigen protein human PD-1 with that of the parent MHPD1-A-7 H Fc.

**Table 10. Binding Activity of Affinity-Matured Nanobody-Fc Fusion Protein to Antigen Protein**

| Antibody Code | EC₅₀ (nM) | Antibody Code | EC₅₀ (nM) |
|---|---|---|---|
| MHPD1-A-7 HFc | 0.149 | MHPD1-A-30 HFc | 0.166 |
| MHPD1-A-15 HFc | 0.134 | MHPD1-A-31 HFc | 0.219 |
| MHPD1-A-16 H Fc | 0.187 | MHPD1-A-32 H Fc | 0.156 |
| MHPD1-A-17 HFc | 0.196 | MHPD1-A-33 HFc | 0.628 |
| MHPD1-A-18 HFc | 0.178 | MHPD1-A-35 HFc | 0.824 |
| MHPD1-A-19 HFc | 0.270 | MHPD1-A-37 HFc | 0.141 |
| MHPD1-A-20 HFc | 0.175 | MHPD1-A-38 HFc | 0.421 |
| MHPD1-A-21 HFc | 0.179 | MHPD1-A-39 HFc | 0.158 |
| MHPD1-A-22 HFc | 0.242 | MHPD1-A-42 HFc | 0.193 |
| MHPD1-A-23 HFc | 0.152 | MHPD1-A-43 HFc | 0.387 |
| MHPD1-A-24 HFc | 0.177 | MHPD1-A-44 HFc | 0.198 |
| MHPD1-A-25 HFc | 0.211 | MHPD1-A-45 HFc | 0.146 |
| MHPD1-A-27 HFc | 0.540 | MHPD1-A-46 HFc | 0.252 |
| MHPD1-A-28 HFc | 0.242 | MHPD1-A-47 HFc | 0.238 |
| MHPD1-A-29 HFc | 2.181 | MHPD1-A-49 HFc | 0.171 |

Some fusion proteins with high affinity to human PD-1 were selected to detect the competitive binding activity with recombinant PD-L1 to human PD-1. 96-well plates were coated with recombinant human PD-L1-mFc (8 µg/mL) at 4°C overnight. The plates were washed three times with PBST. 5% PBS-Milk was added and incubated at room temperature for 1 hr. All the nanobody-Fc fusion proteins were subject to 3-fold gradient dilutions with an initial concentration of 10 µg/mL, added into each well at 30 µL/well, and incubated at room temperature for 1 hr. 0.5 µg/mL of recombinant human PD-1-Fc-Biotin was added into the 96-well plates coated with the antigen at 30 µL/well and incubated at room temperature for 1 hr. The plates were washed with PBST between incubations. Horseradish peroxidase-labelled streptavidin (1:10000) was used for detection, and Graphpad Prism software was used to fit the curve and calculate the IC₅₀ value, as shown in Table 11. The results show that after the affinity maturation, the competitive inhibitory activity of MHPD1-A-15 H Fc against the binding of the antigen protein to the ligand h-PD-L1 was increased as compared with that before the affinity maturation, and the competitive inhibitory activities of multiple fusion proteins are superior to that of the positive control.

**Table 11. Competitive Inhibitory Activity of Affinity-Matured Nanobody-Fc Fusion Protein and Recombinant Human PD-L1 Against Human Recombinant PD-1**

| Antibody Code | IC₅₀ (nM) | Antibody Code | IC₅₀ (nM) |
|---|---|---|---|
| MHPD1-A-7 H Fc | 0.778 | MHPD1-A-32 H Fc | 1.718 |
| MHPD1-A-15 HFc | 0.517 | MHPD1-A-37 HFc | 1.544 |
| MHPD1-A-20 HFc | 1.211 | MHPD1-A-39 HFc | 0.850 |
| MHPD1-A-21 HFc | 1.609 | MHPD1-A-45 HFc | 0.944 |
| MHPD1-A-23 HFc | 1.202 | MHPD1-A-49 HFc | 1.262 |
| MHPD1-A-30 HFc | 1.078 | Pembrolizumab | 1.248 |

## Claims

1. An isolated antigen binding protein binding to the same or overlapping PD-1 epitope with a reference antibody.

2. The isolated antigen binding protein according to claim 1, competing with the reference antibody for binding to PD-1.

3. The isolated antigen binding protein according to any one of claims 1-2, with its binding affinity for PD-1 being substantially the same as the binding affinity of the reference antibody for PD-1.

4. The isolated antigen binding protein according to any one of claims 1-3, having one or more of the following properties:
1) binding to PD-1 with an EC50 of about 11 µg/mL or less in ELISA assay;
2) inhibiting the binding of human PD-L1 to human PD-1 with an EC50 of about 2 µg/mL or less in competitive ELISA assay.

5. The isolated antigen binding protein according to any one of claims 1-4, being capable of binding to PD-1 derived from primates.

6. The isolated antigen binding protein according to claim 5, wherein the primates comprise human and/or monkeys.

7. The isolated antigen binding protein according to any one of claims 1-6, wherein the reference antibody comprises a HCDR3, and the HCDR3 comprises an amino acid sequence as shown in SEQ ID NO: 128.

8. The isolated antigen binding protein according to any one of claims 1-7, wherein the reference antibody comprises a HCDR2, and the HCDR2 comprises an amino acid sequence as shown in SEQ ID NO: 127.

9. The isolated antigen binding protein according to any one of claims 1-8, wherein the reference antibody comprises a HCDR1, and the HCDR1 comprises an amino acid sequence as shown in SEQ ID NO: 126.

10. The isolated antigen binding protein according to any one of claims 1-9, wherein the reference antibody comprises a heavy chain variable region VH, and the VH comprises the HCDR1, HCDR2, and HCDR3, the HCDR3 comprises an amino acid sequence as shown in SEQ ID NO: 128; the HCDR2 comprises an amino acid sequence as shown in SEQ ID NO: 127; and the HCDR1 comprises an amino acid sequence as shown in SEQ ID NO: 126.

11. The isolated antigen binding protein according to claim 10, wherein the VH comprises an amino acid sequence as shown in SEQ ID NO: 132.

12. The isolated antigen binding protein according to any one of claims 1-11, wherein the reference antibody comprises a LCDR3, and the LCDR3 comprises an amino acid sequence as shown in SEQ ID NO: 131.

13. The isolated antigen binding protein according to any one of claims 1-12, wherein the reference antibody comprises a LCDR2, and the LCDR2 comprises an amino acid sequence as shown in SEQ ID NO: 130.

14. The isolated antigen binding protein according to any one of claims 1-13, wherein the reference antibody comprises a LCDR1, and the LCDR1 comprises an amino acid sequence as shown in SEQ ID NO: 129.

15. The isolated antigen binding protein according to any one of claims 1-14, wherein the reference antibody comprises a light chain variable region VL, and the VL comprises the LCDR1, LCDR2, and LCDR3, the LCDR3 comprises an amino acid sequence as shown in SEQ ID NO: 131; the LCDR2 comprises an amino acid sequence as shown in SEQ ID NO: 130; and the LCDR1 comprises an amino acid sequence as shown in SEQ ID NO: 129.

16. The isolated antigen binding protein according to claim 15, wherein the VL comprises an amino acid sequence as shown in SEQ ID NO: 133.

17. The isolated antigen binding protein according to any one of claims 1-16, wherein the reference antibody comprises pembrolizumab or an antigen binding fragment thereof.

18. The isolated antigen binding protein according to any one of claims 1-17, comprising a HCDR3, and the HCDR3 comprises an amino acid sequence as shown in SEQ ID NO: 138.

19. The isolated antigen binding protein according to claim 18, wherein the HCDR3 comprises an amino acid sequence as shown in SEQ ID NO: 142.

20. The isolated antigen binding protein according to any one of claims 18-19, wherein the HCDR3 comprises an amino acid sequence as shown in any one of SEQ ID NOs: 3, 55, 56, 57, 58, 59, 60, 61, 62, 63, 64, 65, 66, and 67.

21. The isolated antigen binding protein according to any one of claims 1-20, comprising a HCDR2, and the HCDR2 comprises an amino acid sequence as shown in SEQ ID NO: 137.

22. The isolated antigen binding protein according to claim 21, wherein the HCDR2 comprises an amino acid sequence as shown in SEQ ID NO: 141.

23. The isolated antigen binding protein according to any one of claims 21-22, wherein the HCDR2 comprises an amino acid sequence as shown in any one of SEQ ID NOs: 2, 42, 43, 44, 45, 46, 47, 48, 49, 50, 51, 52, 53, and 54.

24. The isolated antigen binding protein according to any one of claims 1-23, comprising a HCDR1, and the HCDR1 comprises an amino acid sequence as shown in SEQ ID NO: 136.

25. The isolated antigen binding protein according to claim 24, wherein the HCDR1 comprises an amino acid sequence as shown in SEQ ID NO: 140.

26. The isolated antigen binding protein according to any one of claims 24-25, wherein the HCDR1 comprises an amino acid sequence as shown in any one of SEQ ID NOs: 1, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 41.

27. The isolated antigen binding protein according to any one of claims 1-26, comprising a heavy chain variable region VH, and the VH comprises the HCDR1, HCDR2, and HCDR3, the HCDR3 comprises an amino acid sequence as shown in SEQ ID NO: 138; the HCDR2 comprises an amino acid sequence as shown in SEQ ID NO: 137; and the HCDR1 comprises an amino acid sequence as shown in SEQ ID NO: 136.

28. The isolated antigen binding protein according to claim 27, wherein the VH comprises the HCDR1, HCDR2, and HCDR3, the HCDR3 comprises an amino acid sequence as shown in SEQ ID NO: 142; the HCDR2 comprises an amino acid sequence as shown in SEQ ID NO: 141; and the HCDR1 comprises an amino acid sequence as shown in SEQ ID NO: 140.

29. The isolated antigen binding protein according to any one of claims 27-28, wherein the VH comprises the HCDR1, HCDR2, and HCDR3, the HCDR3 comprises an amino acid sequence as shown in any one of SEQ ID NOs: 3, 55, 56, 57, 58, 59, 60, 61, 62, 63, 64, 65, 66, and 67; the HCDR2 comprises an amino acid sequence as shown in any one of SEQ ID NOs: 2, 42, 43, 44, 45, 46, 47, 48, 49, 50, 51, 52, 53, and 54; and the HCDR1 comprises an amino acid sequence as shown in any one of SEQ ID NOs: 1, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, and 41.

30. The isolated antigen binding protein according to any one of claims 27-29, wherein the HCDR1, HCDR2, and HCDR3 comprises any one group of amino acid sequences selected from:
1) HCDR1: SEQ ID NO: 1, HCDR2: SEQ ID NO: 2, and HCDR3: SEQ ID NO: 3;
2) HCDR1: SEQ ID NO: 1, HCDR2: SEQ ID NO: 42, and HCDR3: SEQ ID NO: 55;
3) HCDR1: SEQ ID NO: 4, HCDR2: SEQ ID NO: 43, and HCDR3: SEQ ID NO: 3;
4) HCDR1: SEQ ID NO: 5, HCDR2: SEQ ID NO: 44, and HCDR3: SEQ ID NO: 3;
5) HCDR1: SEQ ID NO: 6, HCDR2: SEQ ID NO: 45, and HCDR3: SEQ ID NO: 3;
6) HCDR1: SEQ ID NO: 7, HCDR2: SEQ ID NO: 46, and HCDR3: SEQ ID NO: 3;
7) HCDR1: SEQ ID NO: 8, HCDR2: SEQ ID NO: 47, and HCDR3: SEQ ID NO: 3;
8) HCDR1: SEQ ID NO: 9, HCDR2: SEQ ID NO: 46, and HCDR3: SEQ ID NO: 3;
9) HCDR1: SEQ ID NO: 10, HCDR2: SEQ ID NO: 45, and HCDR3: SEQ ID NO: 3;
10) HCDR1: SEQ ID NO: 11, HCDR2: SEQ ID NO: 44, and HCDR3: SEQ ID NO: 3;
11) HCDR1: SEQ ID NO: 12, HCDR2: SEQ ID NO: 43, and HCDR3: SEQ ID NO: 3;
12) HCDR1: SEQ ID NO: 13, HCDR2: SEQ ID NO: 48, and HCDR3: SEQ ID NO: 3;
13) HCDR1: SEQ ID NO: 14, HCDR2: SEQ ID NO: 45, and HCDR3: SEQ ID NO: 3;
14) HCDR1: SEQ ID NO: 15, HCDR2: SEQ ID NO: 47, and HCDR3: SEQ ID NO: 3;
15) HCDR1: SEQ ID NO: 16, HCDR2: SEQ ID NO: 43, and HCDR3: SEQ ID NO: 3;
16) HCDR1: SEQ ID NO: 17, HCDR2: SEQ ID NO: 49, and HCDR3: SEQ ID NO: 3;
17) HCDR1: SEQ ID NO: 18, HCDR2: SEQ ID NO: 46, and HCDR3: SEQ ID NO: 3;
18) HCDR1: SEQ ID NO: 19, HCDR2: SEQ ID NO: 46, and HCDR3: SEQ ID NO: 3;
19) HCDR1: SEQ ID NO: 20, HCDR2: SEQ ID NO: 50, and HCDR3: SEQ ID NO: 56;
20) HCDR1: SEQ ID NO: 21, HCDR2: SEQ ID NO: 46, and HCDR3: SEQ ID NO: 57;
21) HCDR1: SEQ ID NO: 22, HCDR2: SEQ ID NO: 46, and HCDR3: SEQ ID NO: 3;
22) HCDR1: SEQ ID NO: 9, HCDR2: SEQ ID NO: 51, and HCDR3: SEQ ID NO: 3;
23) HCDR1: SEQ ID NO: 9, HCDR2: SEQ ID NO: 46, and HCDR3: SEQ ID NO: 58;
24) HCDR1: SEQ ID NO: 23, HCDR2: SEQ ID NO: 46, and HCDR3: SEQ ID NO: 3;
25) HCDR1: SEQ ID NO: 24, HCDR2: SEQ ID NO: 46, and HCDR3: SEQ ID NO: 59;
26) HCDR1: SEQ ID NO: 25, HCDR2: SEQ ID NO: 46, and HCDR3: SEQ ID NO: 60;
27) HCDR1: SEQ ID NO: 26, HCDR2: SEQ ID NO: 46, and HCDR3: SEQ ID NO: 61;
28) HCDR1: SEQ ID NO: 27, HCDR2: SEQ ID NO: 46, and HCDR3: SEQ ID NO: 3;
29) HCDR1: SEQ ID NO: 28, HCDR2: SEQ ID NO: 52, and HCDR3: SEQ ID NO: 3;
30) HCDR1: SEQ ID NO: 29, HCDR2: SEQ ID NO: 46, and HCDR3: SEQ ID NO: 3;
31) HCDR1: SEQ ID NO: 25, HCDR2: SEQ ID NO: 52, and HCDR3: SEQ ID NO: 62;
32) HCDR1: SEQ ID NO: 9, HCDR2: SEQ ID NO: 50, and HCDR3: SEQ ID NO: 63;
33) HCDR1: SEQ ID NO: 30, HCDR2: SEQ ID NO: 46, and HCDR3: SEQ ID NO: 3;
34) HCDR1: SEQ ID NO: 31, HCDR2: SEQ ID NO: 46, and HCDR3: SEQ ID NO: 3;
35) HCDR1: SEQ ID NO: 32, HCDR2: SEQ ID NO: 53, and HCDR3: SEQ ID NO: 3;
36) HCDR1: SEQ ID NO: 33, HCDR2: SEQ ID NO: 52, and HCDR3: SEQ ID NO: 3;
37) HCDR1: SEQ ID NO: 34, HCDR2: SEQ ID NO: 46, and HCDR3: SEQ ID NO: 64;
38) HCDR1: SEQ ID NO: 35, HCDR2: SEQ ID NO: 46, and HCDR3: SEQ ID NO: 57;
39) HCDR1: SEQ ID NO: 9, HCDR2: SEQ ID NO: 54, and HCDR3: SEQ ID NO: 3;
40) HCDR1: SEQ ID NO: 9, HCDR2: SEQ ID NO: 46, and HCDR3: SEQ ID NO: 65;
41) HCDR1: SEQ ID NO: 36, HCDR2: SEQ ID NO: 46, and HCDR3: SEQ ID NO: 3;
42) HCDR1: SEQ ID NO: 37, HCDR2: SEQ ID NO: 52, and HCDR3: SEQ ID NO: 3;
43) HCDR1: SEQ ID NO: 9, HCDR2: SEQ ID NO: 46, and HCDR3: SEQ ID NO: 59;
44) HCDR1: SEQ ID NO: 38, HCDR2: SEQ ID NO: 54, and HCDR3: SEQ ID NO: 3;
45) HCDR1: SEQ ID NO: 39, HCDR2: SEQ ID NO: 46, and HCDR3: SEQ ID NO: 3;
46) HCDR1: SEQ ID NO: 40, HCDR2: SEQ ID NO: 46, and HCDR3: SEQ ID NO: 55;
47) HCDR1: SEQ ID NO: 41, HCDR2: SEQ ID NO: 46, and HCDR3: SEQ ID NO: 3;
48) HCDR1: SEQ ID NO: 9, HCDR2: SEQ ID NO: 46, and HCDR3: SEQ ID NO: 66;
49) HCDR1: SEQ ID NO: 9, HCDR2: SEQ ID NO: 46, and HCDR3: SEQ ID NO: 67.

31. The isolated antigen binding protein according to any one of claims 1-30, comprising a H-FR1, a C-terminus of the H-FR1 is directly or indirectly linked to an N-terminus of the HCDR1, and the H-FR1 comprises an amino acid sequence as shown in SEQ ID NOs: 68, 144-148, 230 and/or 235.

32. The isolated antigen binding protein according to any one of claims 1-31, comprising a H-FR2, the H-FR2 is located between the HCDR1 and the HCDR2, and the H-FR2 comprises an amino acid sequence as shown in SEQ ID NOs: 69, 149-151, and/or 231.

33. The isolated antigen binding protein according to any one of claims 1-32, comprising a H-FR3, the H-FR3 is located between the HCDR2 and the HCDR3, and the H-FR3 comprises an amino acid sequence as shown in SEQ ID NOs: 70, 152-161, and/or 232.

34. The isolated antigen binding protein according to any one of claims 1-33, comprising a H-FR4, an N-terminus of the H-FR4 is directly or indirectly linked to a C-terminus of the HCDR3, and the H-FR4 comprises an amino acid sequence as shown in SEQ ID NOs: 71, 162, and/or 233.

35. The isolated antigen binding protein according to any one of claims 1-34, comprising a H-FR1, a H-FR2, a H-FR3, and a H-FR4, the H-FR1 comprises an amino acid sequence as shown in SEQ ID NOs: 68, 144-148, 230, and/or 235; the H-FR2 comprises an amino acid sequence as shown in SEQ ID NOs: 69, 149-151, and/or 231; the H-FR3 comprises an amino acid sequence as shown in SEQ ID NOs: 70, 152-161, and/or 232; and the H-FR4 comprises an amino acid sequence as shown in SEQ ID NOs: 71, 162, and/or 233.

36. The isolated antigen binding protein according to any one of claims 1-35, comprising a heavy chain variable region VH, and the VH comprises an amino acid sequence as shown in SEQ ID NOs: 139 and/or 234.

37. The isolated antigen binding protein according to claim 36, comprising a heavy chain variable region VH, and the VH comprises an amino acid sequence as shown in SEQ ID NOs: 143 and/or 236.

38. The isolated antigen binding protein according to any one of claims 36-37, wherein the VH comprises an amino acid sequence as shown in any one of SEQ ID NOs: 72-120 and/or 163-227.

39. The isolated antigen binding protein according to any one of claims 1-38, comprising an antibody or an antigen binding fragment thereof.

40. The isolated antigen binding protein according to claim 39, comprising a single-domain antibody or an antigen binding fragment thereof.

41. The isolated antigen binding protein according to any one of claims 39-40, wherein the antigen binding fragment is selected from the group consisting of Fab, Fab', F(ab)2, Fv fragment, F(ab')2, scFv, di-scFv, VHH, and/or dAb.

42. The isolated antigen binding protein according to any one of claims 39-41, wherein the antibody comprises a chimeric antibody, a humanized antibody, and/or a fully human antibody.

43. The isolated antigen binding protein according to any one of claims 1-42, comprising an amino acid sequence as shown in any one of SEQ ID NOs: 72-120 and/or 163-227.

44. The isolated antigen binding protein according to any one of claims 1-43, further comprising an Fc region of an immunoglobulin.

45. The isolated antigen binding protein according to claim 44, wherein the VH of the antigen binding protein is directly or indirectly linked to the Fc region.

46. The isolated antigen binding protein according to any one of claims 44-45, wherein a C-terminus of the VH of the antigen binding protein is directly or indirectly linked to an N-terminus of the Fc region.

47. The isolated antigen binding protein according to any one of claims 44-46, wherein the VH of the antigen binding protein is fused with the Fc region in frame.

48. The isolated antigen binding protein according to any one of claims 44-47, wherein the VH of the antigen binding protein is linked to the Fc region through a linker.

49. The isolated antigen binding protein according to claim 48, wherein the linker comprises a peptide linker.

50. The isolated antigen binding protein according to any one of claims 48-49, wherein the linker comprises a flexible linker.

51. The isolated antigen binding protein according to any one of claims 48-50, comprising, sequentially from N-terminus to C-terminus, the VH of the antigen binding protein, the linker, and the Fc region.

52. The isolated antigen binding protein according to any one of claims 44-51, wherein the Fc region comprises an Fc derived from IgG1 or an Fc derived from IgG4.

53. The isolated antigen binding protein according to any one of claims 44-52, wherein the Fc region comprises an amino acid sequence as shown in any one of SEQ ID NOs: 121-125 and/or 228-229.

54. A polypeptide, comprising the isolated antigen binding protein according to any one of claims 1-53.

55. An immunoconjugate, comprising the isolated antigen binding protein according to any one of claims 1-53 or the polypeptide according to claim 54.

56. An isolated nucleic acid molecule, encoding the isolated antigen binding protein according to any one of claims 1-53 or the polypeptide according to claim 54.

57. A vector, comprising the isolated nucleic acid molecule according to claim 56.

58. A cell, comprising and/or expressing the isolated antigen binding protein according to any one of claims 1-53, the polypeptide according to claim 54, the immunoconjugate according to claim 55, the isolated nucleic acid molecule according to claim 56 and/or the vector according to claim 57.

59. A method for preparing the isolated antigen binding protein according to any one of claims 1-53 and/or the polypeptide according to claim 54, comprising culturing the cell according to claim 58 under a condition allowing the expression of the isolated antigen binding protein and/or the polypeptide.

60. A pharmaceutical composition, comprising the isolated antigen binding protein according to any one of claims 1-53, the polypeptide according to claim 54, the immunoconjugate according to claim 55, the isolated nucleic acid molecule according to claim 56, the vector according to claim 57, the cell according to claim 58, and optionally a pharmaceutically acceptable adjuvant and/or excipient.

61. A method for detecting the presence and/or content of PD-1, comprising:
administering the isolated antigen binding protein according to any one of claims 1-53 or the polypeptide according to claim 54.

62. A kit, comprising the isolated antigen binding protein according to any one of claims 1-53 or the polypeptide according to claim 54.

63. The kit according to claim 62, comprising instructions for describing the method for detecting the presence and/or content of PD-1.

64. Use of the isolated antigen binding protein according to any one of claims 1-53 or the polypeptide according to claim 54 in the preparation of a kit used in a method for detecting the presence and/or content of PD-1.

65. Use of the isolated antigen binding protein according to any one of claims 1-53 and/or the polypeptide according to claim 54 in the preparation of a drug for preventing and/or treating a disease or a disorder.

66. The isolated antigen binding protein according to any one of claims 1-53, the polypeptide according to claim 54, the immunoconjugate according to claim 55, the isolated nucleic acid molecule according to claim 56, the vector according to claim 57, the cell according to claim 58 and/or the pharmaceutical composition according to any one of claim 60, being used for preventing, relieving, and/or treating a disease or a disorder.

67. The use according to any one of claims 65-66, wherein the disease or disorder comprises a tumor.

68. The use according to claim 67, wherein the tumor comprises a solid tumor.

69. The use according to any one of claims 67-68, wherein the tumor comprises a blood tumor.

70. The use according to any one of claims 67-69, wherein the tumor comprises a tumor associated with the expression of PD-L1.

71. The use according to any one of claims 67-70, wherein the tumor is selected from the group consisting of melanoma, lung cancer, head and neck squamous cell carcinoma, lymphoma, hepatocellular carcinoma, renal cell carcinoma, urothelial carcinoma, colorectal cancer, and breast cancer.

72. A method for preventing and/or treating a disease or a disorder, comprising administering to a subject in need an effective amount of the isolated antigen binding protein according to any one of claims 1-53, the polypeptide according to claim 54, the immunoconjugate according to claim 55, the isolated nucleic acid molecule according to claim 56, the vector according to claim 57, and/or the cell according to claim 58.

73. The method according to claim 72, wherein the disease or disorder comprises a tumor.

74. The method according to claim 73, wherein the tumor comprises a solid tumor.

75. The method according to any one of claims 73-74, wherein the tumor comprises a blood tumor.

76. The method according to any one of claims 73-75, wherein the tumor comprises a tumor associated with the expression of PD-L1.

77. The method according to any one of claims 73-76, wherein the tumor is selected from the group consisting of melanoma, lung cancer, head and neck squamous cell carcinoma, lymphoma, hepatocellular carcinoma, renal cell carcinoma, urothelial carcinoma, colorectal cancer, and breast cancer.

78. A method for inhibiting the interaction between PD-1 and PD-L1, comprising administering to a subject in need an effective amount of the isolated antigen binding protein according to any one of claims 1-53, the polypeptide according to claim 54, the immunoconjugate according to claim 55, the isolated nucleic acid molecule according to claim 56, the vector according to claim 57, and/or the cell according to claim 58.

79. The isolated antigen binding protein according to any one of claims 1-53, the polypeptide according to claim 54, the immunoconjugate according to claim 55, the isolated nucleic acid molecule according to claim 56, the vector according to claim 57, the cell according to claim 58, and/or the pharmaceutical composition according to any one of claim 60, being used for inhibiting the interaction between PD-1 and PD-L1.

80. Use of the isolated antigen binding protein according to any one of claims 1-53 and/or the polypeptide according to claim 54 in the preparation of a drug for inhibiting the interaction between PD-1 and PD-L1.
